# EUROPEAN PATENT APPLICATION

(11) **EP 4 737 551 A2**
(43) Date of publication of application: **06.05.2026**
(21) Application number: 26163823.3
(22) Date of filing: 06.10.2021
(51) Int. Cl.: C12M 1/00

(54) **A METHOD AND SYSTEM FOR CONFIGURING AND/OR SETUP OF A DOWNSTREAM PROCESS FOR PROCESSING A BIOMASS**

(30) Priority: 14.10.2020 EP 20201696
(62) Divisional of application: 21787412.2
(71) Applicant: Sartorius Stedim Biotech GmbH, 37079 Göttingen (DE)
(72) Inventor: LEUPOLD, Marco, 37075 Göttingen (DE); BARROSO ESPACHS, Alexandre, 37079 Göttingen (DE); HUSEMANN, Bernward, 37079 Göttingen (DE); RODENBERG, Michael, 37574 Einbeck (DE); MATUSZCZYK, Jens, 37077 Göttingen (DE); KAMPMANN, Markus, 44149 Dortmund (DE); WORTMEYER, Johannes, 37079 Göttingen (DE); FRIESE, Thomas, 37079 Göttingen (DE); SCHOLZ, Jochen, 37079 Göttingen (DE); LEUTHOLD, Martin, 37079 Göttingen (DE)
(74) Representative: Müller-Boré & Partner Patentanwälte PartG mbB

(57) **Abstract**

The present invention relates to a computer-implemented method, a computer program product and a system for configuring and/or setup and/or controlling of a downstream process for processing a biomass. The method comprises receiving at least one measurement value of at least one feature an upstream process by which the biomass to be processed in the downstream process has been obtained and/or of at least one feature of a downstream process stage or operation; determining at least one downstream process parameter based on the received at least one measurement value; and configuring and/or setup and/or controlling at least one downstream process stage or process operation based on the determined at least one downstream process parameter. The at least one feature includes titer, and wherein the determining of the at least one downstream process parameter comprises determining, based on the received measurement value of the titer, at least one process parameter of a chromatography stage or operation, said at least one process parameter including one or more of a dimensioning, loading volume, cycle time, flow rate and number of chromatography columns. The invention further relates to a respective method and a system for processing biomass.

## Description

The technical field of the present application is methods and systems for configuring, setup and/or control of a downstream process for biomass processing. In particular an aspect of the application relates to an interconnection between upstream and downstream processes for biomass processing and/or between the process stages of a downstream process comprising a plurality of process stages.

A process for processing a biomass to obtain a product (bioprocess) is a process using cells and/or their components to obtain desired products. A bioprocess typically comprises a plurality of different stages or operations, for example cell growth stage, cell harvesting stage, media preparation stage, separation stage, chromatography stage, filter (filtering or filtration) stage, stirring stage, etc.

Generally, a bioprocess may be divided in an upstream process and a downstream process. The upstream process (upstream part of the bioprocess) relates to the first part of a bioprocess, in which the cell culture, microbes, etc. is/are grown. An upstream process typically involves a plurality of steps or stages. For example, an upstream process may comprise culture inoculation, culture growth, fermentation, etc. A downstream process (downstream part of the bioprocess) typically involves processing the biomass produced by the upstream process to obtain a final product meeting certain quality and purity requirements. A downstream process typically involves a plurality of stages, such as for example a plurality of purification stages at which waste, impurities and/or other undesired substances are removed. For example, a downstream process may comprise a primary recovery at which cells and/or debris are removed, separation stage (e.g. by centrifugation), filter stage, chromatography stage, buffer exchange stage, material hold stage, viral inactivation stage, other purification stages, etc. Each of the stages of the upstream and downstream process may comprise a plurality of sub-stages. Each individual stage or operation has one or more process parameters or settings that need to be configured, setup and/or controlled to obtain the required final product.

There are various challenges involved in the configuring or setup up and controlling the different stages of a complex bioprocess. An object of the present invention is to provide an improved methods and systems for configuring, setup and/or control of a bioprocess.

The object is solved by a method for configuring and/or setup and/or controlling of a downstream process for A computer-implemented method for configuring and/or setup and/or controlling of a downstream process for processing a biomass, a respective computer program product, a system for configuring and/or setup and/or controlling a downstream process, a method for processing a biomass and a system for processing a biomass as defined in the claims.

In particular, according to a first aspect of the present disclosure, there is provided a computer-implemented method for configuring and/or setup of a downstream process for processing a biomass, comprising:
receiving at least one measurement value of at least one feature of an upstream process by which the biomass to be processed in the downstream process has been obtained;
determining at least one downstream process parameter based on the received at least one measurement value.

The downstream process and more specifically at least one process stage or process operation of the downstream process is configured and/or setup based on the determined at least one downstream process parameter. Thus, the downstream process is carried out in accordance with the at least determined downstream process parameter.

According to a second aspect of the present disclosure, there is provided a computer-implemented method for controlling of a downstream process for processing a biomass, comprising:
receiving at least one measurement value of at least one feature of an upstream process by which the biomass to be processed in the downstream process has been obtained;
determining at least one downstream process parameter based on the received at least one measurement value;
controlling at least one process stage or process operation of the downstream process based on the determined at least one downstream process parameter.

In particular, the control may be predictive control, based on measured values of the at least one feature of the upstream process.

Examples of the biomass to be processed include bacteria, yeasts, molds, animal cells, plant cells, etc. Further ingredients may include chemical compounds, proteins (such as enzymes), various additives, etc.

The downstream processing of the biomass results in a biopharmaceutical product. Some non-limiting examples of such products include recombinant and non-recombinant proteins, vaccines, gene vectors, DNA, RNA, antibiotics, secondary metabolites, growth factors, cells for cell therapy or regenerative medicine, half-synthesized products (e.g. artificial organs). Various production systems may be used to facilitate the process, e.g. cell based systems such as animal cells (e.g. CHO, HEK, PerC6, VERO, MDCK), insect cells (e.g. SF9, SF21), microorganisms (e.g. E. coli, S. cerevisiae, P. pastoris, etc.), algae, plant cells, cell free expression systems (cell extracts, recombinant ribosomal systems, etc.), primary cells, stem cells, native and gene manipulated patient specific cells, matrix based cell systems.

As mentioned above, an upstream process is a bioprocess by which a cell culture, microbial culture, etc. is grown, thereby producing a biomass that is further processed in a downstream process to obtain a final product meeting the requirements for purity, quality, etc. Typically, each of the upstream and downstream process comprises a plurality of stages, each stage being configured to perform a certain process operation or a group of process operations. For example, an upstream process may comprise one or more of the following stages or unit operations: culture inoculation, culture growth, fermentation, etc.

The downstream process may comprise one or more of the following stages or unit operations primary recovery stage at which cells and/or debris are removed, separation stage (e.g. by centrifugation), filter stage, chromatography stage, buffer exchange, material hold stage, viral inactivation stage, other purification stages, etc. Within the present application, the term "purification process/operation" (also referred to as "clarification process/operation") relates to any process/operation associated with the processing of a biopharmaceutical product, during which at least one fraction of a product undergoing processing is separated from or filtered out of the remaining part(s) of the product. The result is a purified (clarified) intermediate or final product having the target properties. Exemplary purification processes/operations are filtration (such as deep (depth) filtration, virus filtration, virus inactivation, tangential flow filtration, sterile filtration, pre-filtration, etc.), centrifugation, chromatography, etc. Similarly, the term "purification stage" (also referred to as "clarification stage") relates to any process stage, during which at least one fraction of a product undergoing processing is separated from or filtered out of the remaining part(s) of the product. Exemplary separation stages are a filtration stage, deep (depth) filtration stage, virus filtration stage, virus inactivation stage, centrifugation stage, tangential flow filtration stage, sterile filtration stage, chromatography stage, purification stage, etc. The term "filter" used in connection with a purification process, stage or operation relates to the device used in the respective purification process, stage or operation that carries out the separation or filtering out.

Each of the stages of the upstream and downstream process may comprise a plurality of sub-stages. Each individual stage or operation may have one or more process parameters or settings that need to be configured, setup and/or controlled to obtain the required intermediate or final (end) product.

Examples of end products of the downstream process include but are not limited to recombinant or non-recombinant proteins, vaccines, gene vectors, DNA, RNA, antibiotics, secondary metabolites, growth factors, etc.

In the method according to the first and second aspects, at least one feature (upstream feature) of the upstream process by which the biomass to be processed in the downstream process has been obtained may be measured and the respective measurement value or values made available to the downstream process configuration and/or setup and/or control. The at least one upstream feature may be a feature characterizing the cells, contaminants, a product, etc. and/or a process parameter of the upstream process. The at least one upstream feature may be a feature that characterizes any stage, process or operation of the upstream process.

Measurements of the at least one upstream process feature may be performed online or offline. In the context of the present description the term "online" includes also inline and atline measurements. Atline measurements may require more time and more manual intervention than inline measurements. However, atline measurements might not involve the time, the level of analysis and manual intervention required for offline measurements.

Online measurements (such as inline measurements) typically do not require removal of a sample of the product, although in some cases online measurements (such as atline measurements) may also involve removal or diversion of a sample of the product to be produced. Online measurements may be obtained using probes, sensors, or measuring devices placed in the processed product and/or the container holding the processed product (such a bioreactor). Online measurements may be carried out at specified intervals throughout the process and may correspond to process parameters that have a defined confidence interval. Examples of online measurements include pH, conductivity, temperature, culture volume, antifoam concentration, cell density, cell turbidity, cell viability, flow sensor parameter, including sensor quality, spectroscopy data, e.g. indicative of the total protein concentration, metabolite and/or medium concentration, pressure value of a tube and/or bioreactor, etc.

Offline measurements may require a sample of the processed product to be diverted for in-depth analysis. In some cases, offline measurements may be performed once for a batch. Examples of offline measurements include cell viability, total protein concentration obtained by offline measurement or offline sample, lactate dehydrogenase or other enzyme activity, upstream product quality, e.g. of a monoclonal antibody, upstream product aggregation level, product concentration, DNA/HCP concentration, titer, etc.

The measurement value(s) of the at least one upstream feature may be made available to the downstream process configuration and/or setup and/or control. For example, the measured value(s) may be transmitted to a receiving device involved in the downstream process by means of a wireless transmission, via computer network (e.g. over the Internet) or by any other suitable communication means. The receiving device may be a part of a system for configuring and/or setup and/or controlling a downstream process, which itself may be a part of a system for configuring and/or setup and/or controlling the overall biological process (i.e. both the upstream and the downstream process).

Based on the measurement value(s) of the at least one upstream feature, one or more settings, i.e. one or more parameters of the downstream process are determined, for example by a suitable programmed computer system comprising one or more processing units (processors). The computer system may be a part of a system for configuring and/or setup and/or controlling a downstream process (downstream process configuration, setup and/or control system).

The determined downstream process parameter(s) may be stored in a database or other suitable data storage unit. The database may further store additional process parameters or data, such as for example (functional) relations or rules linking the at least one upstream feature and its values and at least one downstream parameter or setting. The functional relations or rules linking the at least one upstream feature and its values and the at least one downstream parameter or setting may be obtained by various means, for example based on past data and/or using mathematical models. For example, to obtain the functional relations various statistical methods may be employed, including, but not limited to various regression analysis methods, machine learning methods, etc.

The stored process parameter(s), optionally together with additional parameters or data and the functional rules, may be used by the downstream process configuration, setup and/or control system to automatically setup, configure and/or control at least one downstream process stage or process operation. The database may be accordingly accessible over a suitable computer network by the respective devices and/or control units.

The database may be implemented as a cloud database, i.e. a database that runs on a cloud computing platform. In other words, the database may be accessible over the Internet via a provider that makes shared processing resources and data available to computers and other devices on demand. The database may be implemented using a virtual machine image or a database service. The database may use an SQL based or NoSQL data model.

The database may provide access control such that some of the process parameters are accessible by a plurality of users of the database or respective control devices and some process parameters are private and only accessible by a limited number of users or one particular user or respective control devices.

In the context of the present description, the term "determining" encompasses any of the following operations: calculating, setting, changing and/or adjusting of at least one downstream process parameter, i.e. of the settings relating to at least one process parameter of the downstream process. The term "receiving" encompasses any of the following operations: obtaining the respective data (measurement value(s) of at least one upstream feature), receiving wirelessly transmitted data, receiving data transmitted over computer networks such as Internet, WLAN, etc., reading data from a permanent or temporary storage unit (such as example database) or receiving or obtaining data by any suitable means. The data may be received via a suitable interface or interfaces of a computer system. Controlling of at least one process stage or process operation (such as centrifugating, filtering, chromatography, pumping, storing, etc.) may comprise controlling of at least one process equipment and/or control device (i.e. actuator) associated with the respective process stage or process operations based on determined downstream process parameters. The at least one control device may be a part of a control system, such as the downstream process configuration, setup and/or control system control system. The controlling may be carried out while the downstream process and more specifically the respective process stage or process operation is performed using respective process equipment.

According to the first and second aspects described above, the downstream process is automatically setup, configured and/or controlled based on measurements of at least one feature of the upstream process by which the biomass to be processed in the downstream process is obtained. Advantages of the proposed method for controlling a downstream process include reducing the number of labor-intensive and error prone manual operations and thus the overall error rate, optimizing the processing time, simplifying the downstream setup and control process, improving the quality of the output products and reducing waste. Further, it is possible to optimally synchronize the upstream and downstream process stages, which enables reduction of idle, non-productive time and increasing the process efficiency.

Still further, the logistic and stock management may be improved.

Various features or characteristics of the upstream process may be measured (for example automatically) and the measured values made available to the downstream process.

For example, the at least one upstream process feature may include a viable cell density, biomass, capacitance, cell viability and/or turbidity. Based on the measured upstream values, at least one downstream parameter setting may be determined. The determining of the at least one downstream process parameter may for example comprise:
selecting a clarification (purification) stage or operation;
determining, based on the received at least one measurement value of the viable cell density, biomass, capacitance and/or cell viability, at least one process parameter of the selected clarification (purification) stage or operation and/or determining a biomass dilution parameter.

The selecting of a clarification (purification) operation may include selecting between a filter stage or operation and a centrifugal stage or operation, wherein:
if a filter stage or operation is selected, the at least one process parameter may include at least one of the following parameters: a type of the filter stage or operation, number of filters, filter area, pressure, flow rate, amount of diatomaceous earth and process time;
if the centrifugal stage or operation is selected, the at least one process parameter may include the process time, process volume and the centrifugal force.

For example, the measured value of the viable cell density (VCD) of the upstream process may be multiplied with the process volume, in order to determine the overall biomass amount to be processed in the downstream process. Based on the determined biomass amount, the amount of biomass that needs to be separated or clarified may be determined. The amount of biomass that needs to be separated or clarified may also be determined based on the measured turbidity or measured biomass or any other suitable measurable characteristic.

Depending on the biomass that needs to be separated or clarified, a suitable separation method and/or system for the cell separation stage may be determined. The separation method and the respective system may be, for example, a filtering method/system and centrifugal separation. Further, among the filtering methods, different types of filtering may be selected such as Deep (Depth) Filtration, Dynamic Body Feed Filtration, etc.

The (functional) relationship or rule linking the biomass that needs to be separated and respective clarification (purification) operation settings (such as the separation method and/or system) may be stored in a database or other suitable storage media and used to configure, setup and/or control the downstream process. For example, with the increasing amount of the biomass that needs to be separated, the following separation methods and/or systems and the respective stages may be selected in this order: Deep filtration, Dynamic Body Feed Filtration, or Centrifugal separation. In addition, other separation settings, such as the number of the filters, the filter area and/or the process time may be determined. Still further, the level of dilution of the biomass to be processed may be determined, in order to facilitate to separation process.

In addition or alternatively, the downstream clarification (purification) operation settings (such as for example, the g-force of the centrifugation stage, the process time, etc.) may be determined based on the cell viability. For example, lower g-force of the centrifugal stage/operation may be selected for cells that should retain high target viability as compared to the g-force of cells that are damaged or apoptotic. In addition or alternatively, the downstream clarification (purification) settings may be determined based on the targeted degree of sedimentation. For example, the higher the targeted degree of sedimentation (independent of the cell viability), the higher the g-force and/or the process time of the centrifugation operation.

In an example, the at least one upstream process feature may include titer. The determining of the at least one downstream process parameter may comprise determining, based on the received measurement value of the titer, at least one process parameter of a chromatography operation, said at least one process parameter including one or more of a dimensioning, loading volume, cycle time, flow rate, number of chromatography columns, column exchange sequence, loading scenario and/or sequence. The (functional) relationships or rules linking the measured upstream titer and the at least one process parameter of a chromatography stage or operation may be stored in a database or other suitable storage unit and used to configure, setup and/or control the downstream process.

A high titer multiplied by the process volume results in a large quantity of product. Generally, the higher the titer, the smaller the volume that is to be loaded in/over a chromatography column in a loading step. Accordingly, with the increase of the upstream titer, in a downstream process, a larger size and/or higher number of chromatography columns and/or a higher number of cycles may be set/configured.

For example, in a batch process, the measured value of the titer may be multiplied by the process volume to determine the overall amount of the product (biomass) to be processed in the downstream process, for example to be purified by protein A chromatography. Based on the determined overall amount of biomass, the chromatography settings (such as the dimensioning or size of the chromatographic column(s), loading volume, etc.) required for processing the biomass within a predetermined time may be determined and set accordingly. Alternatively, for a given size or dimensions of the chromatography column(s) the processing time (e.g. the number of cycles) may be determined and set accordingly. Further, the amount of puffer may be determined and set accordingly.

In case of a continuous process, based on the titer and the volume flow, the number of chromatography columns necessary to ensure the continuous processing of the flow of biomass from the upstream process to the downstream process may be determined and accordingly activated or connected. Further or alternatively, based on the titer, the number of cycles and/or the timing of the chromatography column(s) exchange (e.g. because of the column's aging after a certain number of cycles) may be determined. Further, based on the titer, at least one recipe of the downstream process may be determined (e.g. at least one recipe according to ISA 88). The determination of the recipe may comprise a new recipe's generation or a change or adjustment of an existing recipe. The generation, change or adjustment of a recipe my for example comprise the determination of a volume flow, the loading volume and/or other parameters. The term "recipe" as used in the present application relates to the necessary set of information that uniquely defines the production requirements for a specific product or operational task (see also the definition of the term "recipe" in ANSI/ISA-88.00.01-201o "Batch Control Part 1: Models and Terminology"). Chapter 6, point 6.1, page 55).

In an example, the at least one upstream process feature may include a host cell protein (HCP) and/or DNA concentration. The determining may comprise determining of the at least one process parameter of an anion or a cation exchange chromatography stage or operation, said at least one process parameter including one or more of a volume of the chromatography column and/or membrane adsorber, buffer volume, number of cycles, and process time. For example, with the increase of HCP, the size or volume of the AEX or CEX column may increase.

Further, the consumption amount(s) of one or more media necessary for the downstream processing of the biomass (such as in an anion exchange (AEX) chromatography operation or cation exchange (CEX) chromatography stage or operation may be determined. Based on the determined consumption amount(s), it is possible to automatically control the stock of the respective media and to automatically order additional amounts and/or change the estimated amount(s) of media to be stocked. This considerably reduces the number of labor-intensive and error prone manual operations and the error rate. Further, there it has considerable advantages in terms of logistic and stock management.

The (functional) relationships or rules linking the upstream host cell protein (HCP) and/or DNA concentration and the at least one process parameter of an anion or a cation exchange chromatography stage or operation may be stored in a database or other suitable storage unit and used to configure, setup and/or control the downstream process.

In an example, the at least one upstream process feature may include the type and/or amount of at least one contaminant. The determining of the at least one downstream process parameter may comprise determining, based on the received measurement value of the type and/or amount of the at least one contaminant, at least one downstream process parameter including one of a biomass dilution parameter, a cycle time and processing column size parameter.

The at least one upstream process feature may include for example the amount of antifoam and/or amount of block polymer(s). The determining of the at least one downstream process parameter may comprise determining, based on the received measurement value of the type and/or amount of antifoam or block polymer(s), at least one downstream process parameter including one of a biomass dilution parameter, a cycle time and processing column size parameter.

For example, the type and/or amount of at least one contaminant, such as the amount of antifoam and/or antiblock polymer in the upstream process may affect the viscosity and thus the process features or steps of the downstream process. For example, higher level of antifoam and/or antiblock polymer may require higher parameter settings in the downstream process, such as higher cycle time, processing column(s) size, etc. Further, the information of the amount of antifoam and/or antiblock polymer may be used to determine the level of dilution, wherein the higher the level of the antifoam and/or antiblock polymer in the upstream process, the higher the level of dilution in the downstream process.

Further, antifoam (AF) and antiblock polymer(s) may cause filter blocking. Accordingly, in a downstream process the amount of antifoam and/or antiblock polymer(s) may be kept as low as possible in the individual downstream process steps. This may be achieved for example by dilution and/or the use of a higher number of filters and/or filters with greater surface areas with increased amounts of antifoam and/or antiblock polymer(s).

The (functional) relationships or rules linking the upstream amounts and/or types of contaminants and the at least one downstream process parameter such as dilution, cycle time, processing time, etc. may be stored in a database or other suitable storage unit and used to configure, setup and/or control the downstream process.

In an example, the at least one upstream process feature may include the pH value and/or the amount of at least one pH correctant (puffer capacity). The determining of the at least one downstream process parameter may comprise determining, based on the received measurement value of the pH value and/or the amount of at least one pH correctant, the amount and/or type of at least one pH correctant for use in the downstream process. Generally, for a given process step, there is an optimal pH value or pH range. The higher the amount by which the measured pH value is greater than the optimal or target pH value, the more base/base concentration is needed and the respective downstream process settings can be set or configured accordingly. Analogously, the higher the amount by which measured pH value is lower than the optimal or target pH value, the more acid/acid concentration is needed and he respective downstream process setting may be set or configured accordingly.

For example, the pH value and/or the amount of at least one pH correctant (puffer capacity) of the upstream process may be used to determine the necessary or optimal amount of and/or type of pH correctant(s) for a protein A chromatography unit operation of the downstream process. In an example, a combination of the pH value (basic or ground pH value) and the pH correcant(s) may be used, in order to determine the necessary or optimal amount and/or type of pH correctant(s) for the downstream process.

Based on the determined amount and/or type of at least one pH correctant for the downstream process, the volume, flow rate, timing and other parameters of the delivery of the pH correctant(s) in the downstream process may be automatically setup, configured and/or controlled, for example by controlling the respective pumps and/or puffer reservoirs. Thus, the error rate may be reduced, the preparation time optimized and the process control simplified. Further, the stock management of pH correctant(s) may be improved.

Further, based on the pH value and/or the amount of at least one pH correctant (puffer capacity) of the upstream process, it may be determined whether to start or not start a downstream processing at all. For example, it the measured pH value is not within a certain threshold, the downstream process may be aborted, i.e. not started.

The (functional) relationships or rules linking the upstream pH value and the at least one downstream process parameter, such as the amount of at least one pH correctant, the amount and/or type of at least one pH correctant, etc. may be stored in a database or other suitable storage unit and used to configure, setup and/or control the downstream process.

Various other upstream process features may be measured and the measured values used to determine (e.g. set of change) at least one downstream process parameter:
the at least one upstream process feature may for example include the amount of lipids and/or long epidermal growth factor (EGF) and/or peptides and/or other media, and the determining of the at least one downstream process parameter may comprise determining, based on the received measurement value of the amount of lipids and/or long epidermal growth factor and/or peptides and/or other media, at least one clarification (purification) operation parameter or setting (such as for example the size and/or number and/or loading cycles, etc. of the chromatography columns); and/or
the at least one upstream process feature may include at least one critical quality attribute (CQA), and the determining of the at least one downstream process parameter may comprise determining, based on the received measurement value of the at least one critical quality attribute, whether to start a downstream process; and/or
the at least one upstream process feature may include the process temperature, and the determining of the at least one downstream process parameter may comprise determining, based on the received process temperature, a cooling or heating of process fluid for the downstream process; and/or
the at least one upstream process feature may include turbidity, and the determining at least one downstream process parameter may comprise determining, based on the received measurement value of the turbidity, at least one clarification (purification) operation parameter or setting (such as for example the size and/or number and/or loading cycles, etc. of the chromatography columns, amount of dilution, etc.); and/or
the at least one upstream process feature may include media buffer system and/or capacity, and the determining of the at least one downstream process parameter may comprise determining, based on the received measurement value of the media buffer system and/or capacity, a pH adjustment parameter; and/or
the at least one upstream process feature may include process volume, and wherein the determining of the at least one downstream process parameter may comprise determining, based on the received measurement value of the process volume, dimensioning of the downstream process (the process volume is typically a global parameter that (in conjunction with other measured parameters) may be used to determine the settings and/or functionalities of one or more (for example all) downstream process steps); and/or
the at least one upstream feature may include conductivity, and wherein the determining of the at least one downstream process parameter may comprise determining, based on the received measurement value of the conductivity, a dilution parameter (for example based on the measured upstream conductivity and a predetermined optimal or target conductivity for at least one downstream process stage corresponding measures like amount of dilution to achieve the optimal or target level of conductivity for a given DSP process step may be determined).

The respective (functional) relationship between the measured at least one upstream feature and the at least one downstream parameter may be stored in a database or other suitable storage unit and used to configure, setup and/or control the downstream process.

In an example, the receiving may comprise receiving a plurality of measurement values of respective plurality of features. The at least one parameter of the downstream process is determined based on the received plurality of measurement values. The use of measurement values of a plurality of measured features enables better and more precise configuration, setup and/or control of the downstream process.

Non-limiting examples include at least two of the following features: the amount of biomass or product to be processed (as expressed for example by the wet cell weight or turbidity), viable cell concentration, cell viability, turbidity and particle distribution of the product/biomass to be processed, for example depending on the portion of the larger and smaller particles. For the same level of viability, the number of filters and/or filter area may increase with the increase of the wet cell weight or turbidity and/or the viable cell concentration. Further, the filter area may be determined depending on the particle distribution of the product/biomass to be processed, for example depending on the portion of the larger and smaller particles.

According to a third aspect of the present disclosure, there is provided a computer program product comprising instructions which, when the program is executed by a computer, cause the computer to carry out the method of any the above described aspects and examples.

According to a fourth aspect of the present disclosure, there is provided a system configured to setup and/or configure and/or control a downstream process for processing a biomass to obtain a product. The system comprises at least one processor configured to carry out the method of any of the above described aspects and examples. Accordingly, the above description of embodiments, examples, technical effects and advantages in connection with the above described aspects and examples applies also to the system according to the fourth aspect of the disclosure.

In particular, the system may comprise a receiving device (which may be a part of the at least one processor or connected to the at least one processor) configured to receive at least one measurement value of at least one feature of an upstream process by which the biomass to be processed in the downstream process has been obtained. Further, the system is configured to determine, by the at least one processor, at least one downstream process parameter based on the received at least one measurement value.

Further, the system may be configured to configure and/or setup and/or control, by the at least one processor, at least one process stage or unit process operation of the downstream process based on the determined at least one downstream process parameter. The at least one processor may be a general or special purpose processor. It is also possible to employ a distributed computing system such as a cloud-based computing system. The system for configuring and/or setup and/or control of a downstream process for processing a biomass to obtain a product is also referred to as a downstream process setup and/or control system.

The system for setup and/or configuring and/or control of a downstream process may further comprise a data storage unit for storing the determined at least one at least one downstream process parameter. The data storage unit may be for example a database, such as the database described in connection with the first and the second aspects of the present disclosure.

The system for setup and/or configuring and/or control of a downstream process may further comprise at least one device (actuator) controlled by the processor, wherein the at least one processor and at least one actuator are configured to jointly to realize the determined configuration and/or setup and/or control of the downstream process (i.e. of at least one downstream process stage or operation). The actuator may for example be a valve that allows the connecting or disconnecting of a particular downstream process stage or operation or a group of downstream process stages or operations in the flowpath of the downstream process. In addition or alternatively, the actuator may influence the pressure, temperature, dilution, or other parameters in a certain downstream process stage or operation, a group of process stages or operations or any other units along the process flowpath.

The system for setup and/or configuring and/or control of a downstream process may further comprise one or more probes, sensors, or measuring devices to monitor one or more parameters of the downstream processes. The measurements may be carried out continuously or at specified intervals throughout the downstream process. The measurements may be used by the one or more control devices for controlling at least one piece of equipment associated with the downstream process (e.g. by using a feedback control loop).

Further, the measurements may be used to setup, configure and/or control at least one process parameter of the upstream process by which the biomass to be processed in the downstream process is obtained. In other words, the exchange of measurement data between the upstream and the downstream process may be bi-directional.

According to a fifth aspect of the present disclosure there is provided a method for processing a biomass comprising:
configuring and/or setup of a downstream process for processing the biomass according to the method of any one of the above described aspects and examples;
processing the biomass by using the so configured and/or setup downstream process.

The above description of embodiments, examples, technical effects and advantages in connection with the above described aspects applies also to the method according to the fifth aspect of the present disclosure.

The method may further comprise
performing an upstream process thereby obtaining the biomass to be processed in the downstream process; and
measuring at least one feature of the upstream process.

As described above in connection with the first and the second aspect, the measuring of the at least one feature of the upstream process may be performed online (e.g. inline or atline) or offline.

According to a sixth aspect of the present disclosure, there is provided a system configured to process a biomass, said system comprising:
at least one system for setup and/or configuring and/or control of a downstream process according to the above described fourth aspect and examples, said system comprising at least one processor configured to perform a method for configuring and/or setup and/or control of a downstream process for processing the biomass according to any one of the above aspects and examples;
at least one process (processing stage) configured to process the biomass by using the so configured and/or setup downstream process.

As described above in connection with the first and the second aspect, the at least one process stage may be a filter stage, chromatography stage, stirring stage, hold-up stage, or any other suitable stage. The at least one process stage may comprise respective pieces of equipment for performing the one or more process operations associated with the respective process stage and one or more control devices for controlling the equipment associated with the respective process stage based on a control signal from the at least one processor of the downstream process setup and/or control system.

The system for processing biomass may further comprise one or more probes, sensors, or measuring devices to monitor one or more parameters of the downstream processes in at least one process stage. The measurements may be carried out continuously or at specified intervals throughout the process. The measurements may be used by the one or more control devices for controlling at least one processing equipment associated with the respective process stage (e.g. by using a feedback control loop and/or predictive control). The measuring of the at least one process parameter of the downstream process may be performed inline, online, atline, or offline.

The system may further comprise
at least one process stage configured to carry out an upstream process to thereby obtain the biomass to be processed in the downstream process;
at least one measuring device for measuring of at least one feature of the upstream process.

As described above in connection with the first and the second aspect, the measuring of the at least one feature of the upstream process may be performed online (e.g. inline or atline) or offline. Further, the above description of embodiments, examples, technical effects and advantages in connection with the above described aspects applies also to the system according to the sixth aspect of the disclosure.

The above described principles, methods and devices may also be employed not only to integrate upstream and downstream processes but also to integrate different sub-processes, process stages and/or different devices, systems or units within one type of process, for example within an upstream process or a downstream process.

For example, it is possible to use measurement data/values related to at least one feature or parameter in one particular process stage, device and/or unit to configure, set and/or control another process stage, device or unit, for example another process stage, device or unit which is prior to or subsequent to the one process stage. The process stages may be, for example, cell growing stage, cell harvest stage, stirring stage, media preparation stage, buffer exchange stage, perfusion stage, kSep, bag testing stage, chromatography stage, filtering stage, etc. The measurement data/values relating to one particular process stage, device and/or unit may also be used to control (for example by a feedback control loop) the particular process stage, device and/or unit.

For example, it is possible to use measurement data/values obtained in a chromatography stage and more specifically in one run of the chromatography stage or operation to optimally configure and/or set (i.e. to parametrize) and/or to control a subsequent process stage or operation, such as a virus activating. In such applications, the cyclic operation of a chromatography stage may be challenging from process control perspective. This may be alleviated by the proposed method.

The exchange of data may be bi-directional. Thus, it is possible to use measurement data of a second process stage following a first process stage to configure, set and/or control the first process stage.

Thus, according to a seventh aspect of the present disclosure, there is provided a computer-implemented method for configuring and/or setup and/or control of a process for processing a biomass to obtain a product comprising a plurality of process operations or stages, comprising:
receiving at least one measurement value of at least one feature of a first one of the plurality of process stages or operations;
determining, based on the received at least one measurement value, at least one process parameter of a second one of the plurality of process stages or operations; and
configuring and/or setup and/or controlling the second one of the plurality of process stages or operations based on the determined at least one downstream process parameter.

In particular, the controlling may employ a predictive control technique, based on measured values of the at least one feature of the upstream process.

As described above, the first one of the plurality of process stages or operations may be an upstream or a downstream process stage or operation and the second one of the plurality of process stages or operations may be a downstream process stage or operation. In an example, both the first one of the plurality of process stages or operations may be downstream process stages or operations. Further, the second process stage or operation may be subsequent to the first process stage or operation or may precede the first process stage or operation. Still further, more than one process stage or operation may be configured, setup and/or controlled based on measurement values from one or more other process stages or operations. In other words, there may be more than one first process stages or operation and/or more than one second process stages or operations.

As described above in connection with measurements of upstream features, measurements of the at least one feature of a process stage or operation (upstream and/or downstream) may be performed online or offline and the measurement value(s) made available to the downstream process configuration and/or setup and/or control. The at least one feature may characterize any property of the operation(s) carried out by any process stage or operation or groups of process stages or operations, the product prior to, during or after undergoing processing in any process stage and other process conditions. Exemplary features include, but are not limited to pressure, volume flow, volume flow rate, turbidity, viscosity, product amount and/or concentration, amount and/or concentration of contaminants, cell viability, temperature.

As described above, a downstream process typically comprises a plurality (i.e. at least two, typically more than two) of cascaded downstream process stages or operations, such as for example a plurality of purification stages. The individual process stages or operations may be connected in parallel, sequentially or by a mixture of both parallel and sequential connections. In an exemplary implementation of the first to seventh aspects, the individual downstream process stages or operations are separately connectable and disconnectable, for example by use of respective control device (actuators) such as valves. Thus, the overall downstream process may have a modular structure with individual process stages or operations or groups of proces stages or operations being connectable or disconnectable depending on the at least one process parameter determined based on the at least one measurement value. For example, based on the at least one process parameter, one or more additional process stages or operations (such as one or more purification stages or operations) may be added to or removed from the current process stages or operations constituting the downstream process.

The at least one process parameter may thus include the number and/or type of downstream process stages or operations to be connected or disconnected. The configuring and/or setup and/or controlling may accordingly comprise connecting or disconnecting the determined number and/or type of downstream process stages or operations. Further, any other parameters of the connected downstream stages or operations and/or product stream(s) to and from the downstream stages, such as pressure, dilution, temperature, additives, etc., may be configured, setup and/or controlled based on the at least one determined downstream processing parameter. For example, based on the measured values, at least one parameter characterizing the dimensioning of the determined number and/or type of downstream process stages or operations may be determined and used by the setup, configuration and/or control. The at least one dimensioning parameter may depend on the specific downstream process stage and may, for example, include filter area, filter throughput, retention time, binding capacity, flow rate, amount of additives, process time, process volume, centrifugal force, etc. Thus, one or more downstream stages or operations can be flexibly adapted to the output (for example concentration, cell density, viability, titer, etc.) of the upstream process or of the previous downstream process stages or operations.

In an example, the at least one connectable process stage or operation may be a purification stage or operation, such as a filtration stage (e.g. deep (depth) filtration stage, virus filtration stage, virus inactivation stage, tangential flow filtration stage, sterile filtration stage, pre-filtration stage, etc.), chromatography stage, centrifugation stage, etc. In addition or alternatively, the at least one connectable process stage may be a further process stage, such as temporary or permanent storage stage or operation, stirring stage or operation, etc. During the purifying process, at least one property of the filter and/or of the product prior to, during and/or after undergoing purification processing may be measured. On the basis of the measurement, during the purifying process, further purification stages (e.g. further filters, pre-filters, chromatography columns, etc.) may be dynamically/actively connected or disconnected and/or the properties of the product subject to a particular purification operation may be altered/adapted.

Further, the above description of embodiments, examples, technical effects and advantages in connection with the above described first to sixth aspects apply also to the method according to the seventh aspect of the present disclosure.

According to an eighth aspect of the present disclosure, there is provided a computer program product comprising instructions which, when the program is executed by a computer, cause the computer to carry out the method of the seventh aspect.

According to a ninth aspect of the present disclosure, there is provided a system for configuring and/or setup and/or control of a process for processing a biomass to obtain a product. The system comprises at least one processor configured to carry out the method according to the seventh aspect.

The system according to a ninth aspect of the present disclosure may be configured to in a the same or similar way and comprise the same or similar components as the system described in connection with the first to sixth aspects and examples. For example, the downstream setup and/or control system may further comprise a receiving device to receive the measurement signal from at least one sensor measuring at least one upstream and/or downstream process feature. The system may further comprise at least one sensor for measuring the at least one upstream and/or downstream process feature.

The at least process feature may be measured upstream (i.e. at the input), downstream (i.e. at the output) and/or within a particular process stage or operation. The sensor may, for example measure at least one property of at least one process stage or operation (first process stage) from a plurality of process stages or operations of an upstream or downstream process and/or of a product supplied to the first process stage or output from the at least one first process stage. The measured value(s) is provided to the processor, which may determine at least one process parameter and carry out a configuration, setup and/or control of at least one second process stage on the basis of the received measured value(s).

The system for configuring and/or setup and/or control of a process for processing a biomass may also comprise at least one device (actuator) controlled by the at least one processor with the help of which the determined configuration and/or setup and/or control is realized. The at least one actuator may be for example a valve.

The at least one processor may for example determine, based on at least one measurement value received from a sensor and employing predictive control techniques, how many process stages should be connected and/or how the process stages are to be configured, setup and/or controlled and may generate a respective control signal to the at least one actuator.

The at least one actuator may for example be a valve associated with a particular process stage and the control signal may be a signal to open or close the valve to disconnect or connect the respective process stage. Alternatively or in addition, the control unit may issue a control signal to respective actuators to control the pressure and/or dilution and/or temperature and/or other process properties and properties of the product to be fed to the at least one second process stage. Thus, the overall process may be optimally configured, setup and/or controlled and failures reduced.

The system may further comprise a data storage unit (e.g. a database) for storing the determined at least one at least one downstream process parameter and/or measurement values and/or rules linking measurement values with process parameters; and/or a receiving device for receiving the at least one measurement value and/or at least one sensor for obtaining the at least one measurement value.

Exemplary processors, receiving devices, sensors and other devices such as actuators and data storage units have been described above in connection with the first to sixth aspects and examples, the description of which applies also to the seventh to night aspects.

According to a tenth aspect of the present disclosure there is provided a method for processing a biomass to obtain a product comprising:
configuring and/or setup and/or controlling of a process for processing the biomass according to the method of the seventh aspect of the present disclosure;
processing the biomass by using the so configured and/or setup and/or controlled downstream process.

According to an eleventh aspect of the present disclosure there is provided a system configured to process a biomass to obtain a product, said system comprising:
at least one system for configuring and/or setup and/or control according to the above described ninth aspect and examples, said system comprising a processor configured to perform a method for configuring and/or setup and/or control of a process for processing the biomass according to the seventh aspect of the present disclosure;
at least one process (processing) stage configured to process the biomass by using the so configured and/or setup process.

The above description of embodiments, examples, technical effects and advantages in connection with the above described first to sixth aspects and examples applies also to the seventh to eleventh aspects of the present disclosure. Further, the above described principles may be applied to any level of process granularity, such as for example to sub-stages of a particular process stage.

Advantages of an exchange of data (uni-directional or bi-directional) between different process stages, devices and/or units or between an upstream and a downstream process include but are not limited to:
- optimal time synchronization of the processes in the different process stages, devices and/or units or between the upstream and downstream processes;
- reduction of idle, non-productive periods and increasing of the process efficiency;
- improving of the yield and/or quality of the final products;
- reducing the waste;
- optimizing media consumption and/or storage;
- optimizing the inventory management and logistic.

Further, due to the induvial process adapting (setup, configuration and/or control) and the modular connection and/or control of individual process stages, one or more of the following advantages may be realized:
- Avoiding or reducing of product loss, for example due to blocked filters; avoiding or reducing the necessity of complex and expensive product recovery procedures
- Optimizing the processes in post- and/or pre-connected process stages in the process workflow, such as for example optimizing the pressure range, adapting for capacity tolerances of filters and/or variations in the product properties in the various stages of the process workflow;
- Avoiding over-dimensioning and the related higher costs
- Reduction of the dead volume
- Maintaining and/or adapting the process volume flow through post- and/or pre-connected process stages in the process workflow.

The above advantages of the flexible and modular connection and/or control of downstream process stages or operations apply both to batch and continuous processes. Exemplary continuous processes are perfusion processes that involve a production of a biopharmaceutical product during which the product is discharged continuously or at arbitrary time intervals to the subsequent process stages.

In particular, in case of prior art continuous processes that take place over a long (e.g. more than 8 hours) process time range, it has been often necessary to over-dimension the individual process stages of steps, in order to ensure sufficient capacity over the whole process time. This has a number of disadvantages. For example, since the dead volume is high in relation to the fluid flow, the immediate switching on or activating of the rather large total filter area at the start of the process leads to relatively strong weakening of the product stream. Further, in particular in the start-up phase, the relatively long dwell time within a particular purification stage (such as a filtration stage, chromatography stage, etc.) impedes or complicates the process monitoring by sensors arranged at the output of the purification stage. If process decisions that depend on sensor monitoring at the output of the purification stage need to be taken, this often means that a portion of the product must be discarded (so called dead volume of the purification stage) due to the lack of measurements. This causes higher costs, in particular if disposable technologies (e.g. disposable filters, etc.) are employed.

In addition, the large filter area of a purification stage in relation to the fluid flow leads to insufficiently high input pressure and thus to the poor fluid flow distribution within the purification stage (e.g. within the filter stage). This has negative effects on the efficiency, process stability, reliability and safety, in particular for the chromatographic stages.

Still further, the product stream fed to a particular purification stage or group of stages as well as the material and properties of the filter exhibit product related variations. In conventional downstream processing methods, the variations are accounted for by over-dimensioning the individual purification stages, which, however causes the above mentioned technical problems.

The above described flexible configuration, setup and/or control of a downstream process comprising a plurality of (individually) connectable and/or controllable stages or operations, in particular a plurality of individually connectable and/or controllable purification stages or operations offers a solution of one or more of the above described problems of conventional biological processing methods and provides the above mentioned advantages.

For example, since the exact amount of filter material for a particular overall process may be determined in advance and flexibly changed if needed, an over-dimensioning of the system is not necessary and the disadvantages associated with over-dimensioning may be alleviated or avoided altogether.

Further, due to the adaptive connection and disconnection of process stages and sub-stages (such as purification stages and sub-stages) during the process, it is possible to implement large-scale batch processes in a continuous mode for the first time. The implementation of such large-scale processes with long processing time in a continuous mode would be otherwise not feasible.

Still further, it is possible to dispose of intermediate storage tanks, since due to the active and flexible connection and disconnection and/or control of individual process stages and sub-stages, the volume throughputs of the individual process stages and sub-stages may be balanced.

In addition, in case disposable vessels and components, such as disposable bioreactors, connections, sensors, filter elements, etc. are used, the automatic connection and disconnection of individual process stages and sub-stages can significantly reduce the undesired contaminations, which is important for sterile processes in which such disposable vessels and components are used.

The main application of the processes and systems according to the above described aspects and examples is the purification of biopharmaceutical products. These include cells, cell fragments, compounds on a protein basis (e.g. antibodies, viruses, virus-like particles, RNA, DNA, etc.). Other applications include chemical and food industry, water processing, etc.

These and other aspects will now be described in detail with reference to the following drawings:
**Fig. 1** showing schematically an exemplary system for configuring and/or setup of a downstream process for processing a biomass to obtain a final product;
**Fig. 2** showing schematically another exemplary system for processing a biomass;
**Fig. 3** showing schematically a method for setup, configuration and/control of a biological process based on sensor information;
**Fig. 4** showing a block diagram of an exemplary process for processing a biomass in an exemplary system comprising a bioreactor;
**Fig. 5** showing an exemplary dependency of the filter capacity and filter area on the cell number in a bioreactor;
**Fig. 6A** showing a block-diagram of one large purifications stage;
**Fig. 6B** showing a block-diagram of an exemplary modular purification stage comprising a plurality of smaller connectable purification stages;
**Fig. 7** showing an exemplary dependency of the virus decrease on the differential pressure during the virus filtration for three different monoclonal antibodies;
**Fig. 8** showing a block-diagram of an exemplary pre-purification stage connected to a main purification stage
**Fig. 9** showing a block-diagram of an exemplary purification process with a plurality of membrane absorbers,
**Fig. 10** showing a block-diagram of an exemplary purification process with an intermediate storage tank.

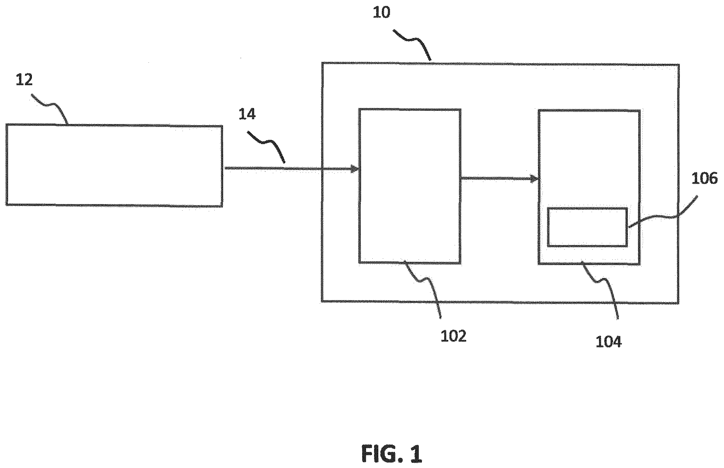

**Fig. 1** shows schematically an exemplary system 10 for configuring and/or setup and/or control of a downstream process for processing a biomass to obtain a final product. The system 10 comprises a receiving device 102 for receiving measurement value(s) 12 of at least one feature of an upstream process (upstream feature) by which a biomass to be processed by the downstream process is obtained. The measurement value(s) 12 of the at least one upstream feature may be transmitted to the receiving device 102 over a suitable communication network 14, such as a computer network, wirelessly or by any other suitable communication means. The measurement value(s) 12 of the at least one upstream feature may be measured and/or transmitted online or offline. In addition or alternatively, the receiving device 102 may be configured to receive at least one measurement value or at least one feature of the downstream process (downstream feature). The measurement value(s) of the at least one downstream feature may be measured and/or transmitted online or offline.

The at least one upstream and/or downstream feature may characterize the biomass or product before, during the processing and/or after processing in any upstream and/or downstream process stage or group of stages, a process parameter of any upstream and/or downstream process stage or group of stages and/or any other relevant process property. As described above, the at least one upstream and/or downstream feature is/are measured by respective sensors, that may be a part of the system 10 for configuring and/or setup and/or control of a downstream process.

Exemplary online upstream features may include any of the following features:
- pH (used for example to set/control a pH parameter of a downstream process);
- conductivity (used for example to set/control a conductivity parameter of a donwstream process),
- temperature (used for example to set/control a temperature parameter of a donwstream process, typically in the range of 4°C to 30°),
- culture volume (used for example to configure/set/control a downstream process, such as diatomeaceos earth cell harvest process),
- antifoam concentration (used for example to configure/set/control a downstream process, for example a high antifoam concentration may be negative for the downstream process),
- cell density, cell culture turbidity, cell viability (used for example to configure/set/control a downstream process, such as diatomeacoes earth process, set the clarification (purification) settings, such as for example centrifugal settings, filter settings, chromatography settings, etc.),
- flow sensor parameter, including sensor quality (used for example to configure/set/control a continuous downstream process, such as a continuous downstream process from a perfusion culture),
- spectroscopy data, e.g. indicative of the total protein concentration,
- metabolite and/or medium concentration;
- pressure value of a conduit (such as tube) and/or bioreactor, etc (typically in the range of 0.1 to 4 bar);
- viscosity in or at the output of a bioreactor (typically in the range of 1 to 50 cP).

Exemplary offline upstream features may include any of the following features:
- cell viability;
- total protein concentration obtained by offline measurement or offline sample;
- lactate dehydrogenase or other enzyme activity;
- upstream product quality, e.g. of a monoclonal antibody;
- upstream product aggregation level;
- product concentration;
- DNA/HCP concentration.

Exemplary downstream features may include:
- turbidity,
- conductivity,
- cell density;
- cell viability;
- viscosity
- pressure;
- flow rate;
- temperature (typically in the range of 4°C to 30°C);
- metabolite and/or medium concentration;
- contaminant concentration;
- protein concentration.

The system 10 comprises further at least one processor 104 (for example constituting or being a part of a computer-supported control unit) configured to determine at least one downstream process parameter based on the received at least one measurement value of at least one upstream and/or downstream feature. The at least one processor 104 is further configured to automatically set or configure and/or control at least one process stage or process operation of the downstream process based on the determined at least one downstream process parameter. The configuring and/or setup and/or control may be realized by issuing a control signal or signals to at least one actuator, which upon receiving the control signal from the at least one processor 104 implements the determined control. The actuators may be for example valves controlled by respective control signals received from the processor 104. The processor 104 further comprises a database 106 for storing the measured values of one or more upstream and/or downstream features and/or predetermined (functional) relationships or rules that link the one or more upstream and/or downstream features with one or more downstream features. The database 106 may be a standalone unit that is linked to the at least one processor 104 via suitable communication link, for example over the internet, WLAN, etc.

Optionally, the processor 104 may be configured to setup, configure and/or control the upstream process based on the received at least one measurement value of the at least one downstream feature.

The setup, configuration and/or control of the downstream and/or upstream process stage may include the influence or adjustment of at least one upstream and/or downstream process parameter, respectively. The at least one downstream and/or upstream process parameter may be a parameter relating to any downstream and/or upstream process stage, respectively.

For example, the at least one processor 104 may determine, based on the measured value of the at least one feature and predetermined parametrization/rules, the type and/or number of process stages (such as purification stages) that are to be connected or disconnected in the downstream process and may generate a respective control signal that opens or closes respective valves associated with each of process stages.

In addition or alternatively, the at least one processor 104 may determine at least one process parameter of process stages subsequent to and/or prior to a first process stage or group of stages and may issue a control signal to respective actuators to control the at least one process parameter in these stages. The at least one parameter may be for example a pressure and/or dilution and/or other properties of the product to be fed to a particular process stage or group of stages, pressure, temperature, etc. within particular process stage or group of stages, etc..

In an example, at least one additional sensor may be provided to measure at least one property at the output of a particular process stage, for example to monitor the efficiency of the purification process. The measured value(s) may be provided to the at least one processor 104 and used to control the particular process stage based on the measured signal from the sensor. Thus, it is possible to further optimize the overall process.

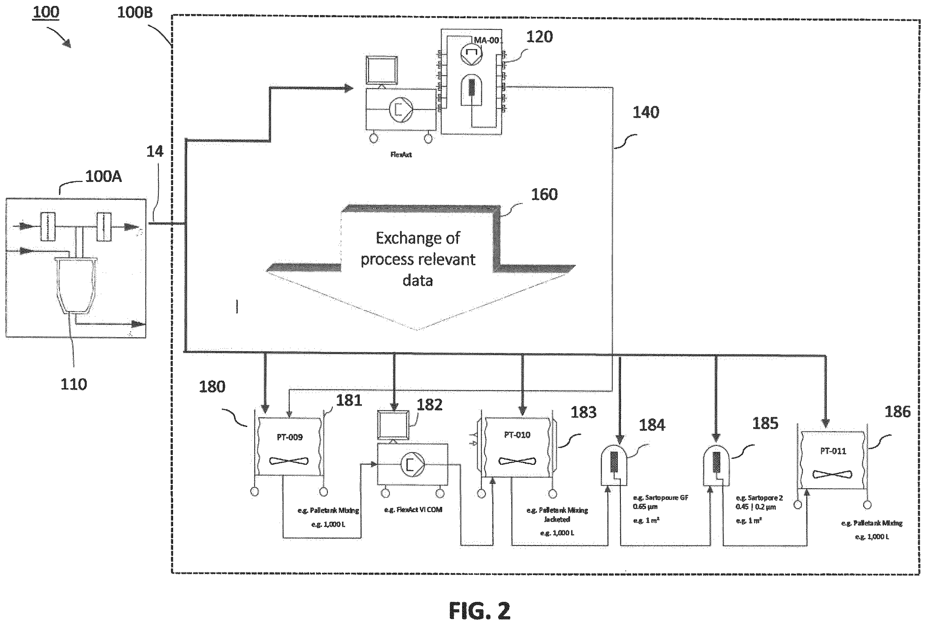

**Fig. 2** shows schematically an exemplary system 100 for processing a biomass to obtain a product comprising a plurality of process stages. The system 100 comprises an upstream processing sub-system 100A and a downstream processing sub-system 100B.

The upstream processing sub-system 100A includes at least one bioreactor 110. The bioreactor 110 may be of any scale, from a small lab-size bioreactor to a very large bioreactor with volumes of about 2000 L and higher. Bioreactors of various sizes are available for example from Sartorius Stedim Biotech. Measurement value(s) of at least one upstream feature of the upstream process(es) carried out by the upstream processing sub-system 100A are transmitted to the downstream processing sub-system 100B over a communication network 14, such as over a computer network, wirelessly or by any other suitable communication means. The transmitted measurement values of the at least one upstream feature may be used to configure or setup the downstream process carried out by the downstream processing sub-system 100B.

The downstream processing sub-system 100B comprises a plurality of process stages. The number, type, size, etc. of the process stages may vary, depending on the specific application. As shown in Fig. 2, one or more of the process stages of the downstream processing sub-system 100B may be configured or setup based on the measurement data received from the upstream processing sub-system 100A.

In the example shown in Fig. 2, the downstream processing sub-system 100B comprises a Protein A chromatography stage 120 followed by a virus inactivation stage 180. The virus inactivation stage 180 comprises a plurality of sub-stages 181-186, each performing a particular sub-process or group of sub-processes. The sub-stage 181 is a stirring stage, e.g. a stirring stage in a 1000 L Palletank^{®} of Sartorius Stedim Biotech. The sub-stage 182 is a low pH virus inactivating stage, such as for example FlexAct^{®} VI of Sartorius Stedim Biotech. The sub-stage 183 is a stirring stage, e.g. in a 1000 L Palletank^{®} of Sartorius Stedim Biotech. The sub-stages 184 and 185 are filtering stages, e.g. by using a Sartopure^{®} GF 0.65 µm filtering unit, the sub-stage 186 is a is a stirring stage, e.g. a stirring stage in a 1000 L Palletank^{®} of Sartorius Stedim Biotech. The process stages shown in Fig. 2 are only exemplary. As mentioned above, it is possible to use other stages or stage combinations, such as other stages of other types, sizes, etc.

Measurement values of at least one feature of the process(es) carried out by one or more of the stages of the downstream processing sub-system 100B (downstream feature) may be obtained and transmitted to other stages of the downstream processing sub-system 100B over a communication network 140, such as a computer network. The transmitted measurement values of the at least one downstream feature may be also used to configure or setup the at least one other stage to which these values are transmitted. The measurement values of at least one upstream feature and/or the measurement values of at least one downstream feature form a part of process relevant data 160 exchanged within the downstream processing sub-system 100B and used to configure or setup and/or control one or more of the downstream process stages. The exchange of process relevant data may be, for example, managed by a respectfully configured processing unit.

For example, the exchanged process relevant data 160 may comprise one or more of features of the Protein A chromatography stage 120 that may be measured and transmitted over a communication network (e.g. a computer network) 140 to the virus inactivation stage 180 and/or other downstream stages. The one or more features of the Protein A chromatography stage 120 may include the number of chromatography cycles, progress in time, volume of one chromatography cycle, puffer strength or amount, type of puffer, pH of the eluate, etc. Based on the received feature(s), one or more process parameters of the inactivation stage and/or other downstream stages are configured or setup.

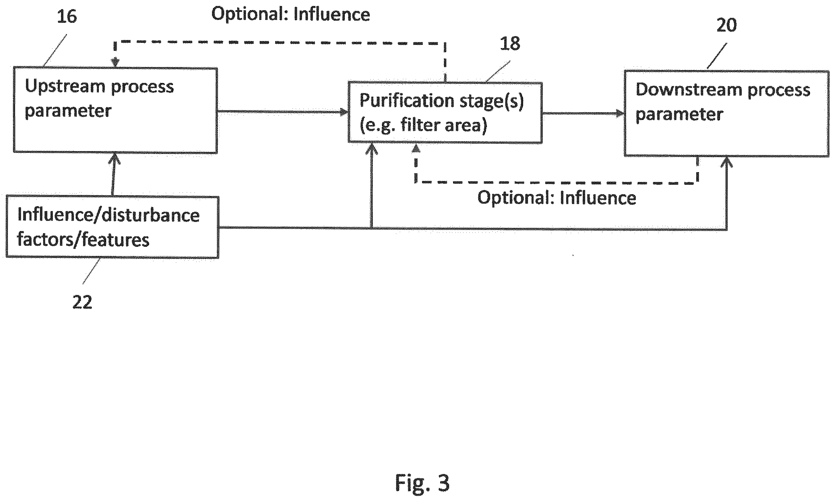

**Fig. 3** shows schematically an exemplary method for setup, configuration and/or control of a biological process, using for example the systems shown in Figs. 1 and 2.

As described above, the biological process comprises an upstream process, during which a biomass (such as a cell culture) is produced, and a downstream process, during which the biomass produced by the upstream process is processed to a final product meeting target quality and purity requirements. The downstream process comprises at least one purification stage 18 during which the input biomass is purified, for example by removing (separating or filtering out) at least a fraction of undesired substances.

At least one feature of the upstream process (upstream feature) and/or of the downstream process (downstream feature) may be measured and the measured value, optionally together with other measured values, may be used to influence (e.g. setup, configure and/or control) at least one upstream process parameter and/or at least one downstream process parameter. The upstream and/or downstream measured features 22 used to influence the downstream and/or the upstream process are together referred to as influence/disturbance factors/features. As described above, such features may be measured online and offline by suitable sensors.

Typical features are described above and include, but are not limited to, pressure (typically in the range of 0.1 to 4 bar), volume flow rate, turbidity, viscosity (typically in the range of 1 to 50 cP), product concentration (typically in the range of 1 to 200 g/L), cell concentration, undesired substances (such as impurities, contaminants, etc.) and their composition (for example the fraction of DNA, proteins, aggregates, etc.), temperature (typically in the range of4°C to 30°C), filter performance (e.g. pressure increase or decrease, retention time, etc.). Typical disturbance features that are monitored may include, deviations of the concentration of the target molecules or other target substances, of the amount and/or composition of undesired substances (such as impurities, contaminants, etc.) in intermediate and/or end products, of the temperature, of the filter performance (e.g. pressure increase or decrease, deviations of the filter capabilities, retention time, deposition rate, binding capability, flow rate, etc.).

In an example, at least one measured value of at least one upstream feature may be used to influence at least one downstream parameter, for example to setup, configure or control at least one parameter of at least one downstream purification stage. Similarly, at least one measured value of at least one downstream process feature may be used to influence at least one upstream parameter 16 and/or at least one downstream parameter 20. For example, as described above, at least one measured value of at least one feature of a first downstream process stage may be used to influence (e.g. setup, configure or control) at least one process parameter of a second downstream process stage and/or of the first downstream process stage itself (e.g. using a feedback control loop and/or predictive control techniques). The second downstream process stage may be a stage that follows the first process stage or precedes the first process stage. The same principle may be applied when the first and/or the second process stage is an upstream process stage.

Typical process parameters that are configured, setup and/or controlled are the number and/or type of purification stages (such as filter stages, etc.), filter area, duration of a purifying operation (such as filter, chromatographic and/or centrifugal operation), centrifugal force, pressure, amount of dilutant or other additives, pump delivery rate, temperature, etc.

The configuration, setup and/or control may be such as to reach and/or maintain (optimal) target characteristics, for example by reducing or compensating deviations of the concentration of the target molecules or other target substances, of the amount and/or composition of impurities in intermediate and/or end products, of the temperature, of the filter performance (e.g. pressure increase or decrease, deviations of the filter capabilities, retention time, deposition rate, binding capability, deposition rate, flow rate, etc.).

Below are some examples of process parameter for setup, configuration and/or control based on measured values of upstream and/or downstream features using for example the systems shown in Figs. 1 and 2 and the method shown in Fig. 3.

**Table 1** shows exemplary upstream process (USP) features and the respective downstream process (DSP) parameters that may be set/configured or adjusted/controlled. In Table 1, category 1 denotes cells or cell cultures, category 2 denotes products, category 3 denotes impurities and category 4 denotes process control parameter. Different levels of priority may be attributed to the individual measured USP features depending on their importance, for example 1: high priority, 2: medium priority and 3: low priority.

| **Priority** | **Category** | **On-/ Offline** | **USP Parameter** | **Utilization in DSP** | **Description** |
|---|---|---|---|---|---|
| 1 | 1 | Online or offline | Viable cell density (VCD), Biomass, Capacitance | Clarification (purification) settings (e.g. filter area, type, amount of diatomaceous earth, etc.) | VCD may be multiplied with the process volume to obtain the total amount of biomass. Based on the total amount of biomass, the amount of the cell mass that needs to be separated may be determined. Based on the determined amount of the cell mass that needs to be separated and predetermined settings (stored for example in a database), the preferred method/system for the cell separation step may be selected. For example, with the increase of the cell mass the following separation methods may be selected in this order: Deep (Depth) Filtration, Dynamic Body Feed Filtration, Centrifugation. In addition, the number of filters and/or filter area may be set/configured. |
| | | | | | Further, based on the upstream VCD, biomass and/or capacity the amount of dilution of the biomass to be processed in the downstream process may be determined and the downstream process accordingly setup, configured and/or controlled. |
| 1 | 1 | Offline | Viability | Clarification (purification) settings (e.g. filter area, type, amount of diatomaceous earth, etc.), centrifugation g-force | See e.g. Table 3 below |
| 1 | 2 | Online or offline | Titer | Dimensioning, loading of capture, loading volume, cycle time and number, etc. | 1. Batch-Prozess: The total product amount that is to be purified or cleared in a DSP process (for example by Protein A Chromatography) may be determined by multiplication of the titer and the process volume. Based on the total amount of product, the chromatography process parameters such as the dimensioning of the chromatography column(s) to complete the process within a predetermined time may be determined. Alternatively, the process time (and thus the number of cycles) and/or the puffer amount for a predetermined column dimensioning/size may be determined and the chromatography process accordingly configured. |
| | | | | | 2. Continuous Prozess: Based on the titer and the volume flow/volume flow rate the number and/or dimensioning of chromatography columns necessary to ensure continuous flow and processing from the USP to the DSP may be determined. Further or alternatively, the number of cycles, the timing of the column exchange (e.g. due to the aging of the columns after a certain number of cycles) may be determined. Still further, it is possible to generate and/or adjust the recipe based on the measured USP titer (e.g. by setting or adjusting the loading volume, the volume flow rate, etc.). |
| | | | | | See also e.g. Table 2 below |
| 3 | 3 | Offline | Host Cell Protein (HCP) | Dimensioning of AEX/CEX | Generally, the higher the amount of HCP, the larger the size of the AEX/CEX column. |
| 3 | 3 | Offline | DNA | Dimensioning of AEX/CEX | Based om the DNA concentration the dimensioning of the anion exchange chromatography operation (AEX) or the cation exchange chromatography operation (CEX), such as for example the volume of the column and/or the membrane adsorber, number of cycles, process time, etc., may be determined. Further, the determined number and/or amount of consumed media and/or equipment may be automatically compared with available/stocked media and/or equipment and, if needed, additional media and/or equipment ordered. |
| 3 | 3 | Online | Amount of antifoam | Corresponding counter measures like dilution, number of filters, etc. | Depending on the amount of antifoam (AF), a dilution parameter and/or the filter setting (e.g. number of filters) may be determined and set, in order to keep the amount of antifoam as low as possible in the downstream process steps. |
| 3 | 3 | Offline | Amount and/or type of lipids | Dimensioning of the chromatography column(s) | Generally, the more lipids, the greater the size and/or number and/or loading cycles, etc. of the chromatography columns. |
| 3 | | Offline | Amount and/or type of block polymers | Corresponding counter measures like dilution and/or increasing the number of filters | Depending on the amount of block polymer(s), a dilution parameter and/or the filter setting (e.g. number of filters) may be determined and set, in order to keep the amount of block polymer(s) as low as possible in the downstream process steps. |
| 3 | 4 | Online | Amount and/or type of pH correctants (acid/base) | Correction to optimal pH/binding conditions | A combination from the pH value (basic-pH-value) und pH correctants (puffer capacity) may be used to determine the amount and/or type of pH correctants that are necessary, in order to optimally set/configure the pH value of the DSP process steps or stages (such as (Protein A Chromatography). |
| | | | | | See also e.g. Table 5 below |
| 2 | 4 | Online | pH value | Correction to optimal pH/binding conditions | A combination from the pH value (basic-pH-value) und pH correctants (puffer capacity) may be used to determine the amount and/or type of pH correctants that are necessary, in order to optimally set/configure the pH value of the DSP process steps or stages (such as (Protein A Chromatography). |
| | | | | | See also e.g. Table 5 below |
| 2 | | Online | Conductivity | Corresponding counter measures like dilution | Corresponding counter measures like dilution to set an optimal or target level of conductivity for a given DSP process step. The feature "conductivity" may be also be measured for a given downstream process step and used to configure a subsequent downstream process step. |
| 1 | 2 | Offline | Critical Quality Attribute (CQA) | Go/noGo for DSP | If critical quality attribute(s) is/are not fulfilled, the downstream process is terminated (no Go). If critical quality attribute(s) is/are fulfilled, the downstream process starts or continues (Go). |
| 3 | 3 | Offline | Other media / process components (long EGF, peptides, etc.) | Dimensioning of the chromatography column(s) | Generally, the more other media or process components (such as long EGF, peptides, etc.), the greater the size and/or number and/or loading cycles, etc. of the chromatography columns. |
| 1 | 4 | Online | Process temperature | Optimizing cooling/heating of process fluid for DSP | Configuring the temperature settings. |
| | | | | | see e.g. Table 6 below |
| 1 | 3 | Online or offline | Turbidity | clarification (purification) settings (filter area, type, etc.) | The measured turbidity may be an indicator of the amount of biomass (e.g. cell mass) that needs to be separated in a downstream process. Based on the amount of biomass that needs to be separated and predetermined settings (stored for example in a database), the preferred method/system for the cell separation step may be selected. For example, with the increase of the cell mass the following separation methods may be selected in this order: Deep (Depth) Filtration, Dynamic Body Feed Filtration, Centrifugation. In addition, the number of filters and/or filter area may be set/configured. Further, based on the measured turbidity, the amount of dilution of the biomass to be processed in the downstream process may be determined and the downstream process accordingly setup, configured and/or controlled. |
| 3 | 4 | Offline | Media buffer system and capacity | pH adjustment in DSP | Adjustment of the pH settings in the downstream process |
| 1 | 4 | Online or offline | Process volume | Dimensioning of DSP, etc. | The process volume is a global parameter that may be used when determining one or more other parameters as explained above. Thus, the process volume may be used to determine the settings and/or functionalities of one or more (for example all) downstream process steps. |

Not all of the USP features listed in Table 1 need to be taken into account when configuring the DSP process. For example, only USP features with high priority or USP features with high and medium priority may be considered. In general, the configuration of at least one DPS process stage or operation is carried out automatically based on at least one of the upstream features listed in Table 1.

Below are some further examples of downstream process configuration based on upstream features.

### Purification of biomass

In an example, the at least one measured property may include the viable cell concentration, the viability and/or the total wet cell weight in or at the output of a bioreactor used in an upstream process or in or at the output of an intermediate storage tank. Alternative to the total wet cell weight, the turbidity of the bioreactor content may be measured (e.g. online or offline). The turbidity is a summary indicator/parameter related to the cells that are alive, the dead cells and cell fractions.

The viable cell concentration may, for example, be measured online by a capacitance sensor.

Alternatively, it is possible to measure the viable cell concentration offline. The total wet cell weight may be measured by respective weight scales. The turbidity may be measured by a respective turbidity sensor. In addition, the at least one measured feature may include the average cell diameter/size and/or the particle size distribution in or at the output of a bioreactor used in an upstream process or in or at the output of an intermediate storage tank.

Based on the measured values, the purification method(s) and/or the type and/or number of purification stages may be determined and respectively configured or controlled as described above. For example, based on the measured values, the cell separation method and the specific process parameters (such as type and/or number of filters) may be determined.

Below is are non-limiting examples of process parameter settings based on exemplary measured features:
First exemplary process parameter settings:
Measured features at the output of a bioreactor:
Bioreactor volume 120 L
viable cell concentration (vcd) (Chinese Hamster Ovary): 13*10^6 cells/mL
Viability 89%
Wet cell weight (wcd) 60 g/L
Turbidity: 1700 NTU
Average cell diameter 19 µm

### Selected separation stages

Filtration by means of two-stage deep (depth) filtration and subsequent sterile filtration, in order to first separate the large cells and then the small particles):
First stage: Deep (Depth) Filter: 3x Sartoclear DL90 cassettes (each 0,8 m² filter area, retention rates 15 µm | 2 µm)
Second stage: Deep (Depth) Filter: 2x Sartoclear DL20 cassettes (each 0,8 m² filter area, retention rates 0,8 µm | 0,4 µm)
Sterile filter: 2x Sartopore2 XLG, size 0 (each 0,52 m² filter area, pore size 0,8 µm | 0,2 µm)

In an example, all of the determined number of filter stages may be connected or activated at the same time Alternatively, the additional filter stages may be connected one after the other, a new filter stage being connected/activated, if a certain condition(s) is reached. For example, a new filter stage may be connected or activated, if a particular pressure limit at or after a particular filter stage or group of filter stages is reached, if the turbidity after a particular filter stage or group of filter stages is higher than a particular threshold, and/or if the increase of the turbidity after a particular filter stage or group of filter stages is higher than a particular limit (a sharp or sudden increase of the turbidity may be an indication of a particle breakthrough), etc.

For example, for a given measured viability, the number of number of filter stages may be increased with the increase of the wet cell weight and/or viable cell concentration. For example, for a given measured viability, the number of filter stages may be increased if the wet cell weight becomes higher than a certain threshold value (e.g. 90 g/L) and/or the viable cell concentration becomes higher than a certain threshold value.

Further, for a given measured wet cell weight, the number of filters in the first deep (depth) filtering stage may be increased with the increase of the viable cell concentration. For example, for a given wet cell weight, the number of filters in the first deep (depth) filtering stage may be increased, if the measured viable cell concentration becomes than a particular threshold value.

In addition, for a given cell concentration, the number of filters in the second deep (depth) filtering stage and/or in the sterile filtering stage may be increased with the increase of the wet cell weight and/or the turbidity. For example, for a given cell concentration, the number of filters in the second deep (depth) filtering state and/or in the sterile filtering stage may be increased if the wet cell weight and/or turbidity becomes higher than a predetermined limit.

Still further, for known particle size distributions, the filter area may be determined depending on the portion of the larger and smaller particles.

### Second exemplary process parameter settings

Measured features at the output of a bioreactor:
Turbidity of the cell culture, typically in the range of 1500 to 2500 NTU;
Wet cell weight (wtc) of the cell culture, typically in the range of 6% to 8%;
Viable cell density (vcd) of the cell culture, typically in the range of 15 - 20 mln cells/mL.

### Cell separation configuration and settings

For cell separation, three different filter stages may be used, wherein the filter areas or filter capacities of the three stages are in a predetermined ratio, for example approximately 2:1:0.75 or any other suitable ratio.

The respective filter areas of the individual filter stages are determined depending on the volume of the cell suspension to be processed. For example, the first filter stage may have a filter capacity expressed by the ratio of the volume to filter area of approximately 50 L/m². The filter capacities of the second and third filter stages are determined according to the predetermined ratio.

The first filter stage may be a deep (depth) filter stage employing for example a deep (depth) filter with two different retention layers (e.g. having retention rate of 15 µm and 2 µm). The second filter stage may be a finer deep (depth) filter stage employing for example a deep (depth) filter with two different retention layers (e.g. having retention rate of 0.8 µm and 0.4 µm). The third filter stage may be a sterile filter stage employing a sterile filter with two membranes having different pore sizes (e.g. of 0.8 µm and 0.2 µm).

It is possible to employ other number of filters and/or filters of other filter areas, if for examples filters with above described properties do not exist.

If the measured turbidity is higher than 2500 NTU (for example if the measured turbidity is about 3000 NTU), and the wet cell weight and/or the viable cell density are in the above mentioned ranges (see "Measured features at the output of a bioreactor"), the filter area, for example of the second and/or third filter stage, may be increased. Typically a turbidity higher than 2500 NTU, wherein the above mentioned wet cell weight and/or the viable cell density are in the above mentioned ranges, is an indication that a large number of small particles cannot not be retained/filtered out in the first filter stage and should be accordingly processed in the next filter stage or stages. As a rule, the higher the measured turbidity the higher the total filter area.

The relations between the measured features, such as the relation wet cell weight/viable cell density and/or the relation turbidity/wet cell weight and/or the relation volume/wet cell density, may be also used to first determine or select the type of purification stage (centrifugation stage, filter stage, chromatographic stage, etc.) and then to determine the configuration/settings of each of the selected purification stages
The above described three filter stages and filters are exemplary and a different number and/or types of filter stages and filters may be used. For example, if the cells suspension to be purified predominantly includes intact (living) cells and few damaged cells, it is possible to reduce the number of filter stages and filters used. For example, it is possible to employ two filter stages, wherein one of them is a deep (depth) filter stage instead of the above described three filter stages.

The above described principles also apply to other purification stages, such as for example other types of filter stages, chromatographic stages, etc.

In another example, the measured upstream feature may be for example titer. A high titer multiplied by the process volume results in a large quantity of product. Generally, the higher the titer, the smaller the volume that is laded in/over a chromatography column in a loading step. Accordingly, with the increase of the titer in a USP process, in a DSP process, a larger size and/or higher number of chromatography columns and/or a higher number of cycles may be set/configured. Further, the column exchange sequence, loading scenario and/or sequence can be appropriately configured.

**Table 2** shows exemplary setup/configuration of the downstream process (DSP) based on the measured titer during the upstream process (USP).

**Table 2**

| **Upstream Process** | **USP Feature(s)** | **DSP Process parameters** |
|---|---|---|
| 50 L process with 5 g/L product (Fed-Batch toward the process end) | 250 g product | Example: 1,5 L Protein A chromatography column, 6 cycles |
| 50 L process with 10 g/L product (Fed-Batch toward the process end) | 500 g product | Example: 3L Protein A chromatography column, 6 cycles (in case of fast processing) or 1,5L Protein A chromatography column with 12 cycles (in case of slower but more cost-effective process) |
| Perfusionprocess with 0.5 - 4 vvd (25 - 200L per day) with constant product concentrations of 1 g/L | 25 - 200 g product over 24 hours | DSP process parameters that may be set/configured include for example one or more of: chromatography column size and/or number, cycles. |
| Perfusionprocess with 0,5 - 4 vvd (25 - 200L per day) with fluctuating product concentration | Fluctuating product amount over 24 hours | DSP process parameters that may be set/configured include one or more of: chromatography column size and/or number, cycles, column exchange sequence/scenario (e.g. one column being loaded, one being used for purification according to a column backup principle), adaptive column selection and loading scenario/sequence depending on the process characteristics |

Additionally or alternatively the measured upstream feature may be cell viability. **Table 3** shows exemplary setup/configuration of the downstream process based on the measured upstream cell viability. Generally, based on the measured/target cell viability, the clarification (purification) operation settings (such as, for example, the g-force of the centrifugation stage) may be determined. As a general rule, the higher the targeted viability at the end of the centrifugation, the lower the g-force of the centrifugation stage.

In addition or alternatively, the clarification (purification) settings (such as for example the g-force of the centrifugation stage, duration of centrifugation, etc.) may be selected based on the targeted degree of sedimentation. For example, the higher the targeted degree of sedimentation (independent of the cell viability), the higher the g-force and/or the longer the duration of centrifugation.

**Table 3**

| **USP Feature(s)** | **DSP Process parameters** |
|---|---|
| Transfer of the measured viability | Depending on the target requirements for the cell viability a g-force of the centrifugal stage/operation may be set/configured, e.g. a low g-force range (e.g. up to 300 N x g), a middle range g-foce (e.g. from 300 N x g to 3000 N x g) or a high g-force range (e.g. higher than 3000 N x g) . For example a centrifugal stage/operation with a low g-force (e.g. up to 300 N x g) may be selected for cells that should retain high viability. The middle range g-force may be selected in case there is no need to retain high target cell viability and the high range g-force may be selected in case of strong sedimentation and/or for centrifugal efficiency in case of damaged (apoptotic) cells. |

Additionally or alternatively the measured upstream feature may be (viable) cell density or concentration. Generally, the higher the measured (viable) cell density or concentration, the greater the filter area and/or the number of filters and/or the number of filter cycles, etc. This also applies regarding the measured biomass: the larger the amount of measured biomass, the greater the filter area and/or the number of filters and/or the number of filter cycles, etc.

**Tables 4A** and **4B** show exemplary setups/configurations of the downstream process based on the measured upstream cell density or concentration. In particular, Table 4A shows exemplary selection of the downstream cell separation method for CHO cells based on the measured upstream cell density or cell concentration. Table 4B shows exemplary configuration/setup of the employed filter for CHO cell separation based on the measured upstream cell density or cell concentration.

**Table 4A**

| **USP feature(s)** | | **DSP processing parameter** |
|---|---|---|
| *Viable cell density (VCD) [* 10^6*/*ml]* | *Moist Biomass [g*/*l]* | *Cell separation method* |
| < 10 | < 50 | Deep (Depth) filtration + Sterile filtration |
| 10 - 25 | 50 - 150 | Dynamic Body Feed Filtration + Sterile filtration |
| > 25 | > 150 | Centrifugation + Deep (Depth) filtration + Sterile Filtration |

**Table 4B**

| **USP Feature(s)** | | **DSP Process parameters** | | | | |
|---|---|---|---|---|---|---|
| *Volume [l]* | *Moist biomass [g*/*l]* | *Separation method* | *Filter Surface area [m²]* | *Filter Specification* | *Sterile filter surface area [m²]* | *Sterile filter Specification* |
| 200 | 50 | 2-stage Deep (Depth) Filtration - Sterile Filtration | 6,4 | 1. Stage: 5x Sartoclear DL90 Cassettes; | 1,6 | Sartopore2 XLG, Size 2 |
| | | | | 2. Stage: 3x Sartoclear DL20 Cassettes | | |
| 200 | 80 | Dynamic Body Feed + Sterile Filtration | 0,63 | 3x Sartoclear Dynamic Cassettes + 4,5 kg Kieselgur (diatomaceous earth) | 0,8 | Sartopore2 XLG, Size 1 |
| 500 | 80 | Dynamic Body Feed + Sterile Filtration | 1,47 | 7x Sartoclear Dynamic Cassettes + 10,5 kg Kieselgur (diatomaceous earth) | 2,4 | Sartopore2 XLG, Size 3 |
| 500 | 120 | Dynamic Body Feed + Sterile Filtration | 2,1 | 10x Sartoclear Dynamic Cassettes + 15 kg Kieselgur (diatomaceous earth) | 2,4 | Sartopore2 XLG, Size 3 |

Additionally or alternatively the measured upstream feature may be pH. **Table 5** shows exemplary configuration/setup of the downstream process based on the measured upstream pH. The pH configuration/setup depends on the pH requirements (target pH) of the specific process stage. If the pH is within the targeted range, no further adjustment is needed. If the pH deviates from the targeted range, an addition of pH correctants (acid or base type) may be required. The higher the deviation of the pH from the targeted pH, the higher the amount of pH correctants.

**Table 5**

| **Downstream Process** | **USP Feature** | **DSP Process parameter(s)** |
|---|---|---|
| Antibodyprocessing | pH in the range of 6.8 to 7.1 | Carrying over the USP pH value to the DSP process. Generally, no further adjustment of the DSP processs is needed, since the binding capacity is good for this pH range |
| Cell separation (e.g. Kieselgurfiltration with pH Shift) | pH in the range of 6.8 to 7.1 | Increasing acidity to reach a pH in the range of 5.0 -to 5.5. pH. Based on the USP pH value, it is possible to determine the acid amount and/or concentration necessary to reach the target DSP pH value depending on the DSP process (e.g. depending on the puffer capacity of the medium) and set/configure the DSP process accordingly. |

Additionally or alternatively the measured upstream feature may be temperature. **Table 6** shows exemplary configuration/setup of the downstream process based on the measured upstream temperature.

**Table 6**

| **Downstream Process [Correct?]** | **USP Feature(s)** | **DSP Process parameters** |
|---|---|---|
| Temperature of the culture medium | Temperature of the bioreactor (at the end of the process or at the subsequent cooling step, e.g. from | Pre-heating or pre-cooling of the receiving tank, in particular in case of temperature instable products and/or in order to avoid interactions in case of puffers. |
| | 37°C to 5°C | |

The settings, including the (functional) relationships or rules linking the upstream features and downstream parameters in the above Tables 1 to 6 may be stored in the database 106 and used to setup, configure and/or control the downstream process.

At least one of the upstream features listed in the above Tables 1 to 6 may also be measured in a particular stage of the downstream process and used to set of configure a subsequent stage or stages of the downstream process. One exemplary feature that may be measured in a downstream process stage and used to configure a subsequent downstream process step is conductivity. Other exemplary features are pH, pressure, temperature, cell density, turbidity, cell viability, flow rate, metabolite and/or medium concentration, contaminant's concentration, protein concentration, etc.

As described above various other upstream and downstream features may be measured and used alone or in combination to setup/configure and/or control the downstream process. In the following, further examples of downstream process control will be described in more detail.

### Example 1: Cell separation after a bioreactor

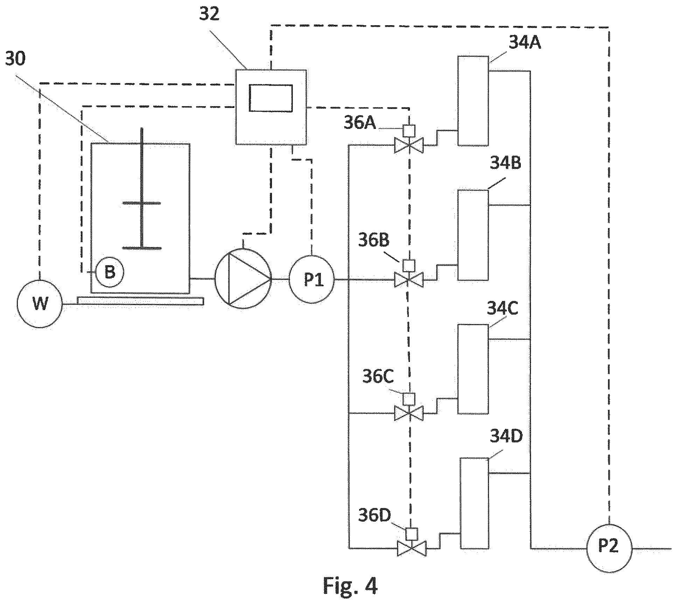

**Fig. 4** shows a block diagram of an exemplary process for processing a biomass in an exemplary system comprising a bioreactor. The process comprises a cell separation stage after the biomass growth stage in a bioreactor.

The system comprises a bioreactor 30, a control unit 32 (as a part of or constituting the downstream setup, configuration and/or control system) comprising at least one processor. Further, the system comprises a group of purification stages 34A to 34D for cell separation arranged downstream of the bioreactor 30. The purification stages 34A to 34D may be for example filtering stages, chromatography stages, centrifugations stages, etc. In the example shown in Fig. 4 the purification stages 34A to 34D are connected in parallel. Other connection patters (such as serial connection or a combination of a parallel and serial connection) are also possible.

Each of the purification stages 34A to 34D can be connected or disconnected in the process flowpath via a respective actuator 36A to 36D (e.g. a valve). Each of the actuators 36A-36D is connected to and controlled by the control unit 32. For example, each of the actuators 36A to 36D may be opened or closed based on a control signal from the control unit 32, thus disconnecting or connecting the respective purification stage 34A to 34D.

The control unit 32 is further connected to and receives measurement signals from a plurality of sensors. The plurality of sensors may, for example, include sensors for determining a concentration of a particular substance or ingredient and/or biomass. The sensors for determining the concentration of an ingredient may for example be spectroscopical sensors (e.g. near infrared sensors, Raman sensors, absorption sensors, etc.). The sensors for determining the biomass may for example be turbidity sensors, impedance sensors, capacity sensors, etc.

Typical features and disturbance features that may be measured and monitored by one or more sensors are the features described for example in connection with Figs. 1 to 3. In particular, typical features measured by the sensors include, but are not limited to:
Upstream: pressure (typically in the range of 0.1 to 4 bar), volume flow rate, turbidity, viscosity (typically in the range of 1 to 50 cP).

Typical disturbance features that are monitored by respective sensors include, but are not limited to deviations of the concentration of the target molecules or other target substances, of the amount and/or composition of impurities in the input, intermediate and/or end product(s), of the temperature, of the filter performance (e.g. deviations of the pressure, filter capabilities, filter etc.), etc.

Based on the measured results, the control unit 32 may determine whether and how many additional purification stages need to be connected and/or the settings of each purification stage, to ensure optimal purification stage capacity and issues the respective control signals to the actuators 36A-36D. Thus, it is possible to reduce or avoid the product losses or complex product reclaiming processes.

In the example shown in Fig. 4 the sensors include:
W a weight sensor (e.g. weight scale), configured to measure the weight of the biomass contained in the bioreactor 30, i.e. the weight of the biomass to be processed in the downstream process;
B a sensor for measuring at least one property of the biomass in or at the output of the bioreactor 30, such as for example a turbidity sensor;
P1 a pressure sensor arranged upstream of the group of purification stages 34A-34D for measuring the pressure at the input to the group of purification stages or the pressure at the output of the bioreactor 30. Typically the pressure at the output of the bioreactor is in the range of 0.1 to 4 bar;
P2 a pressure sensor arranged downstream of the group of separation stages 34A - 34D for measuring the pressure at the output of the group of purification stages or at the input of a process stage (not shown) subsequent to the group of purification stages 34A - 34D.

The measurement signals from these sensors may be transmitted to the control unit and used to configure, setup and/or at least one process parameter, in particular at least one parameter of the downstream process.

In particular, the turbidity sensor B (or other suitable sensor) measures the cell number in or at the output of the bioreactor 30. The weight scales W measures the bioreactor mass or volume that is to be processed (e.g. purified) in the downstream process. In the downstream process, a plurality of purification stages 34A - 34D (e.g. filter stages) for cell separation that are connectable in parallel is provided. Based on the measured signals from the turbidity sensor and the weight scales and predetermined parametrization or rules (for example stored in a database), the control unit 32 determines the number of the purification stages 34A - 34D to be connected and the respective control signals. Based on the control signals, respective valves 36A - 36D associated with each of the purification stages are controlled, for example opened or closed to connect or disconnect the respective purification stage 34A - 34D. The control unit 32 (together with respective actuators) may further control other properties of the product stream to the respective purification stages 34A - 34D, such as for example the flow rate, pressure, temperature, etc. based for example on the measurement signals from the sensors W, B and P and optionally from other sensors such as volume flow rate sensor, viscosity sensor, temperature sensor, etc.

For example, the control unit 32 may optionally further monitor with the help of further pressure sensors, flow sensors, flow rate sensors, viscosity sensors or other suitable sensors arranged prior to and/or after each purification stage 34A - 34D and/or or prior to and/or after the group of purification stages 34A - 34D the level of blocking of a particular purification stage or the group of purification stages. Based on the measured results, the control unit 32 may determine whether and how many additional purification stages need to be connected and/or the settings of each purification stage, to ensure optimal purification stage capacity and issues the respective control signals to the actuators 36A-36D. Thus, it is possible to reduce or avoid the product losses or complex product reclaiming processes.

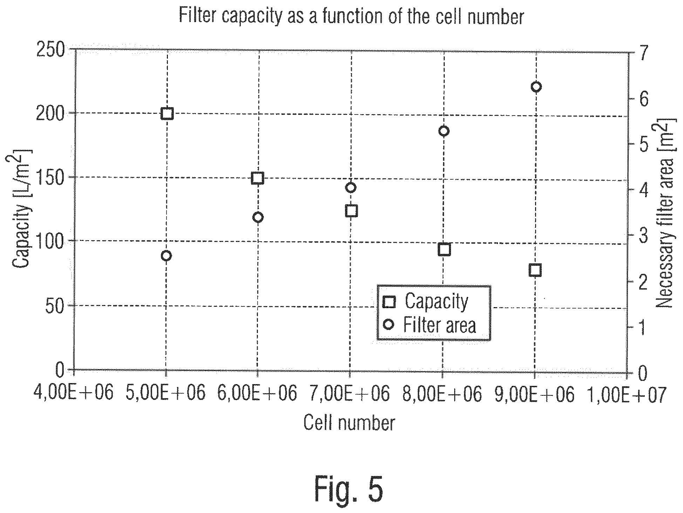

**Fig. 5** shows an exemplary dependency of the filter capacity and filter area on the measured cell number in a bioreactor, wherein the x-coordinate shows the measured cell number in the bioreactor and the y-coordinate the needed filter capacity in units of [L/m²] and the needed filter area in [m²]. The dependency may be stored in a suitable form in a database or other data storage unit connected to or being a part to the control unit 32 and be used to setup, configure and/or control the downstream process and in particular the purification stages 34A - 34D.

Due to the selective connection and/or disconnection of individual purification stages, only the filter area necessary to realize smooth and efficient purification processing may be connected/activated at a particular time. As described above, this prevents over-dimensioning and the resulting disadvantages. For example, the dead volume of the purification process and the resulting high product loss may be reduced or eliminated. In a continuous process, it is possible to quickly achieve a constant volume flow for the subsequent purification stage and the amount of time the product to be processed is retained /processed in a particular purification stage may be significantly reduced.

The control of the purification processes/stages carried out with the help of the control unit 32 may further include determination and control steps for control of further process parameters or devices, such as pump output (pump delivery rate), stirring rate, retention time in an intermediary tank, etc. The control may be based on measurement signals received from one or more of the above mentioned sensors or any suitable further or alternative sensors.

As described in connection with Fig. 3, the setup, configuration and/or control of the plurality of purification stages (such as their number, type and/or other settings) and optionally any further process stages may be such as to reach and/or maintain (optimal) target characteristics, for example by reducing or compensating deviations of the concentration of the target molecules or other target substances, of the amount and/or composition of impurities in the input, intermediate and/or end product(s), of the temperature, of the filter performance (e.g. deviations of the pressure, filter capabilities, filter etc.), etc.

In the above example, four connectable purification 34A - 34D are shown. The number of connectable purification stages is, however, not limited to four and may be higher or smaller than four.

### Example 2: Reduction of the dwell time within a sterile filter stage

As described above, the employment of one purification stage or module having a large filter area in may cause, in particularly in case of low volume flow of the product, blocking of one part of the filter area, while another part is still not being used. After the blocking of one part of the filter area, the product to be purified may start flowing to the other part that has been previously not been used. Since, however, the blocked part remains in the respective purification stage for a relatively long time until the overall purification process is completed, a breakthrough of undesired substance(s) that is/are to be removed or filtered out may occur. This is a significant problem, in particular for sterile filtering processes, where are breakthrough of the bacteria or other similar substances that are to be filtered may occur.

Due to the division of the overall filter area provided by one purification stage into a plurality of connectable modules or purification stages with smaller filter areas, it is possible to add or connect the individual purification stages only when they are needed, for example if a blocking of a particular purification stage is detected. This increases the efficiency of the overall process. Further, it is possible to remove or disconnect a blocked purification stage and thus avoid or prevent a breakthrough of undesired substance(s).

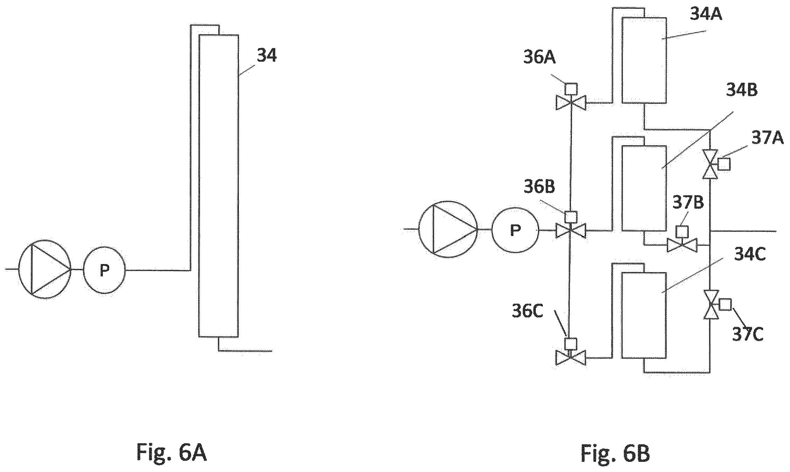

**Fig. 6A** shows schematically one large purification stage 34 with a given (large) filter area and **Fig. 6B** an exemplary division of the purification stage 34 shown in Fig. 6A into a plurality of purification stages 34A, 34B and 34C with smaller filter areas. Each of the plurality of purification stages 34A - 34C is individually connectable or disconnectable by means of respective actuators 36A - 36C and 37A - 37C, wherein the actuators 36A - 36C are arranged at the inputs of the respective purification stages 34A - 34C and actuators 37A - 37C are arranged at the outputs of the respective purification stages 34A - 34C. The actuators 36A - 36C and 37A - 37C are connected to a control unit (not shown), such as the control unit 32 described above. The actuators 36A - 36C and 37A - 37C are controlled by control signals from the control unit, in the manner described above. Thus, it is possible to actively and flexibly connect and disconnect the individual purification stages 34A - 34C and realize various connection patterns.

As described in connection with Figs. 3 to 5, the number of purification stages that are to be activated at a given time of the downstream process and/or the filter area provided by them may be determined and controlled by the control unit based on a number of measured features, in order to reach and/or maintain (optimal) target characteristics. Typical measured features, on which the number of purification stages at a given time is determined are pressure, volume flow, volume flow rate turbidity, viscosity, temperature, etc., that may be measured upstream and/or downstream. Typical monitored disturbance features include deviations of the concentration of the target molecules or other target substances, of the amount and/or composition of impurities in intermediate and/or end products, of the temperature, of the filter performance (e.g. pressure increase or decrease, deviations of the filter capabilities, retention time, etc.), etc.

### Example 3: Determination of optimal operating point for virus filtration

The effect of the operating pressure on the virus filtration is generally known: depending on the operating pressure, the virus filtration/retention capabilities may change (see e.g. Biotechnol Prog. 2017 Sep;33(5):1294-1302). In an example, the operating pressure before (i.e. upstream of) a virus filtration stage or a group of virus filtration stages may be measured by a suitable pressure sensor and based on the measured pressure value, a control unit (such as the above described control unit 32) may issue a control signal to add or remove (i.e. connect or disconnect) at least one additional virus filtration stage. In particular, the control unit may be configured to carry out a control of the filtration stages such that the operation pressure is maintained within a prescribed optimal range by and upon the connection or disconnection of virus filtration stages (such as for example in Fig. 6B). This is particularly advantageous for quasi-continuous processes, since for such processes the total filter surface that needs to be provided is relatively large, however initially, no sufficient pressure difference can be realized due to the relatively low flow rates.

Alternatively or in addition to the pressure, the number and/or properties or settings of the (additional) virus filtration stages may be determined based on other measured features described in connection with Fig. 3. Typical measured features include, but are not limited to:
Upstream: Pressure (typically in the range of 0.1 to 4 bar), volume flow, volume flow rate, viscosity (typically in the range of 1 to 50 cP), temperature (typically in the range of 4°C to 30°C);
Downstream: Pressure, temperature, turbidity, impurities, flow rate, etc.

Typical monitored disturbance features include deviations of the concentration of the target molecules or other target substances, of the amount and/or composition of impurities in intermediate and/or end products, of the temperature, of the filter performance (e.g. pressure increase or decrease, deviations of the filter capabilities, retention time, etc.), etc.

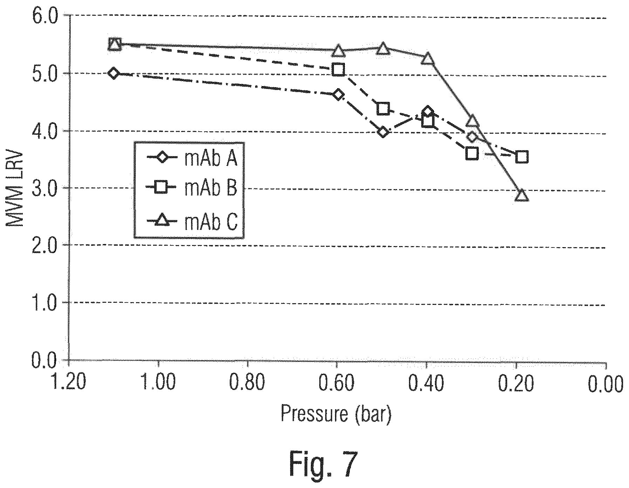

**Fig. 7** shows an exemplary dependency on the virus decrease as a function of the differential pressure during the virus filtration for three different monoclonal antibodies (mAb A, mAb B and mAb C). In Fig. 7 the x-coordinate shows the differential pressure in units of [bar] and the y-axis shows the Log Reduction Value of Minute virus of mice (Log [concentration of virus in the filtrate divided by virus concentration in feed stream]). The dependency may be stored in a suitable form in a database or other storage unit and be used to setup, configure and/or control the downstream process and in particular the virus filtration stages.

### Example 4: Connection of pre-filters, for example for sterile filtration

In an example, pre-stages (such as e.g. pre-filter stages) may be used, in order to increase the capacity of the employed actual main purification stage or group of purification stages (e.g. filter stage(s)). The main purification stage may be for example a sterile filtration stage and a pre-filter may be connected upstream of the main sterile filtration stage. The output or capability of the pre-stages, such as pre-filter stages. may vary due to a variation of the filter material and/or variations of the fed in product solution. This causes a case-to-case variation of the result of the pre-filter stage, in particular for continuous processes.

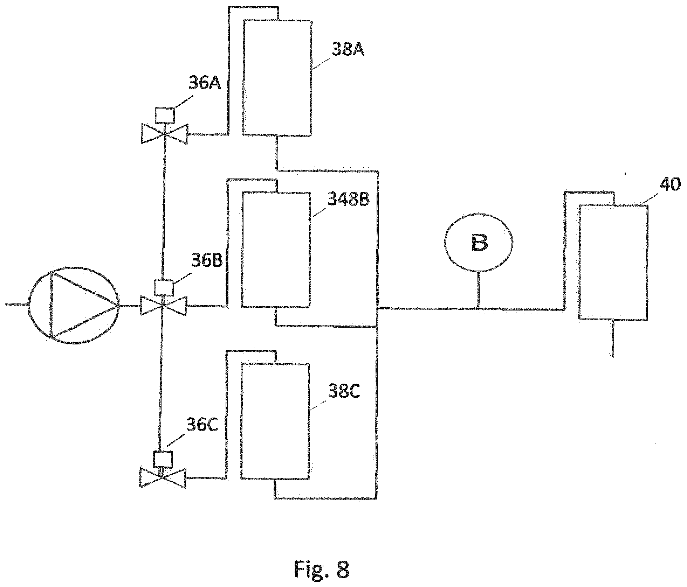

**Fig. 8** shows a block diagram of exemplary pre-purification stages 38A - 38C (e.g. pre-filter stages) connected to a main purification stage 40 (e.g. a main filter). Each of the pre-purification stages 38A - 38 B is connected to a respective actuator 36A - 36C, which is controlled by a control unit (not shown), such as for example the above described control unit 32). The actuators 36A - 36C may be for example valves that may be opened or closed upon receiving a control signal from the control unit, thus disconnecting or connecting the respective pre-purification stages 38A-38C as they are needed. A sensor B at the output of the group of pre-purification stages monitors the biomass/product at the output of the group of pre-purification stages. The sensor B may be a turbidity sensor or any other suitable sensor.

In an example, the quality of purification (e.g. filtration) of the pre-purification stage(s) is monitored by sensor B, for example by measuring the turbidity or other suitable parameters of the filtrate. If the measured turbidity, etc. is above a predetermined value, the control unit issues a control signal to connect (i.e. add) an additional pre-purification stage (e.g. an additional pre-filter stage). The additional pre-purification stage may be connected in series or in parallel to the other pre-purification stage or stages. Thus, it is possible to maintain the turbidity within a pre-determined range and to secure or improve the performance of the main purification stage or stages.

Typical measured features, on which the control unit may configure, setup and/or control the pre-filter stages may include the pressure and/or the volume flow rate measured upstream and turbidity measured downstream. Typical monitored disturbance features include deviations of the concentration of the target molecules or other target substances, of the amount and/or composition of impurities in intermediate and/or end products, of the temperature, of the filter performance of the main filter stage and the pre-filter stage(s) (e.g. pressure increase or decrease, deviations of the filter capabilities, retention time, etc.), etc.

### Example 5: Control of a purification stage Flow-Through-Polishing with a membrane adsorber

Flow-Through-Polishing is a chromatographic filtration step, at which the target value passes through the filter medium and undesired substances are retained by the filter due to chromatographic interaction.

In an example, the chromatographic filtration step is realized by a plurality of adsorber modules (each corresponding to a separate purification stage). The plurality of adsorber modules may be connected and disconnected as needed.

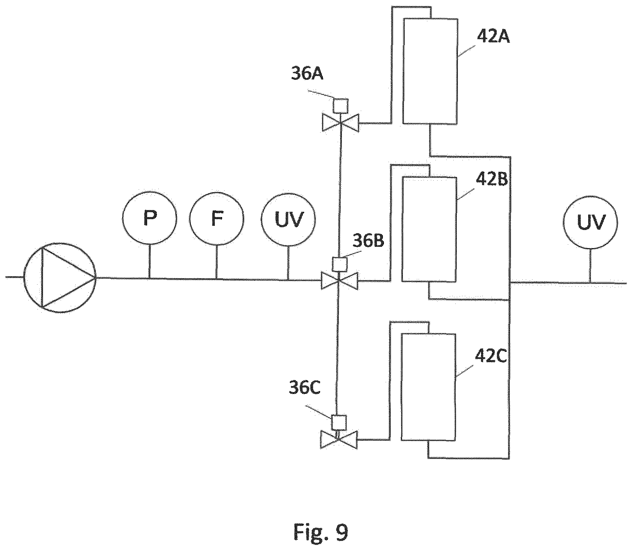

**Fig. 9** shows a block-diagram of an exemplary purification process with a plurality of membrane absorbers 42A - 42C, each connected to a respective actuator 36A - 36C. Each actuator 36A - 36C is controlled by a control unit (not shown), such as for example the above described control unit 32. For example, the actuators 36A - 36C may be valves that may be opened or closed upon a control signal from the control unit, thus disconnecting or connecting the respective membrane absorber 42A - 42C. At the input of the group of membrane absorbers, the following sensors are connected:
P: pressure sensor;
F: flow sensor
UV: UV sensor, e.g. at 280 nm wavelength to detect the concentration of a molecule.

At the output of the group of membrane absorbers 42A - 42C, a sensor UV for measuring the concentration of a molecule is connected.

The measured values by the individual sensors are transmitted to the control unit that uses them to control at least one downstream process parameter. For example, the control unit may generate a control signal to connect or disconnect at least one additional membrane absorber.

The following example relates to a sequential switch on/connection of adsorber modules, thus for example, increasing the number of absorber modules from n to n + 1 or decreasing the number of absorber modules from n to n-1.

As described above, in case of continuous processes with a long processing time (e.g. perfusion processing), it is disadvantageous to immediately switch on or connect a purification stage that provides the total adsorptive capacity, since this causes high dead volume, insufficient feed pressure for achieving a homogeneous distribution of the product that is to be processed, and large output variations due to variations of the product flow and/or filter material.

To reduce and/or avoid the above mentioned disadvantages, the sensor-controlled system may be used as follows:
If the pressure measured at the input of the group of membrane absorbers 42A - 42C is lower than a first pressure which is necessary for assuring homogeneous flow through the process stages, the control unit issues a control signal to reduce the number of membrane absorbers from n to n-1 by disconnecting one of the membrane absorbers. The respective membrane absorber may be disconnected by means of the respective actuator (e.g. a valve) upon receiving a control signal from the control unit. If the measured pressure exceeds a second pressure necessary for assuring homogeneous flow, an additional membrane absorber may be connected, thus increasing the number of membrane absorbers from n to n + 1. Of course, it is possible to connect or disconnect more than one membrane absorber at a time. It is also possible to connect and disconnect membrane absorbers depending on measured properties of the product volume flow and/or viscosity, in order to compensate for variations of the product volume flow and/or viscosity due for example to product and/or filter variations.

Below is a non-limiting exemplary configuration and/or control of a continuous processing method:
- From a perfusion bioreactor with a 500 L volume 500 L of output product solution need to be continuously processed in a downstream process;
- The volume flow rate of the fluid that needs to be continuously filtered is about 0.35 L/min.
- The product concentration is 10 g/L, i.e. per day about 5 kg of product need to be processed
- In a purification stage "Polishing" it is possible to process 5 kg of product per L membrane adsorber (this is for example the capacity of Sartobind Q of Sartorius Stedim). This means that per day a minimum volume of 1 L membrane is needed (corresponds to 3.6 m² Sartobind).
- The recommended flow rate is 5 MV (MV: membrane volume) per minute, in particular in order to assure homogeneous flow. In case of Sartobind Q the recommended flow rate is approximately 5 L/min. The recommended flow rate is many times higher than the volume flow rate of the fluid to be processed (which is approximately 0.35 L/min).
- In order to meet the recommended flow rate, a membrane adsorber with approximately 70 mL total membrane would be necessary.
- The proposed connection of individual connectable membrane absorbers with smaller membrane volumes guarantees that the target process parameters (e.g. volume flow rate and total capacity) can be reliably and efficiently met. Further, since the dead volume is small, it is possible to achieve an early process monitoring at the output of the separation stage or group of separation stages. For example:
   ▪ 1L membrane has approximately 1.3 L dead volume, i.e. at least 4.6 min processing time until the filtrate reaches the output of the respective separation stage and the respective sensor arranged at the output
   ▪ 0.07 membrane has approximately 0.11 L dead volume, i.e. the filtrate reaches the output of the separation stage and the sensor arranged at the output already after approximately after 0.5 min process time.

In the above example, instead of membrane absorbers 42A - 42C other purification stages or modules, such as other filter stages, chromatography stages, etc. may be employed.

Further, in addition or alternatively to the above mentioned upstream and/or downstream features, one or more of the following features may be measured and the measured values used by the control unit to configure, setup and/or control the membrane absorbers. Typical measured features include:
Upstream: Pressure (typically in the range of 0.1 to 4 bar), Volume flow rate, Viscosity (typically in the range of 1 to 5 cP), temperature (typically in the range of 4°C to 30°C,
amount and/or composition of impurities (e.g. fraction of DNA, proteins, aggregates, etc.);
Downstream: amount and/or composition of impurities (e.g. fraction of DNA, proteins, aggregates, etc.).

Typical monitored disturbance features include deviations of the concentration of the target molecules or other target substances, of the amount and/or composition of impurities in intermediate and/or end products, of the temperature, of the filter performance (e.g. binding capacity, Flow performance, etc.), etc.

### Example 6: Adapting of the separation stages in connected processes employing intermediate storage tanks

According to an example, there is provided a sensor-controlled system comprising connected process stages and intermediate storage tanks (i.e. intermediate storage stages). The use of intermediate storage tanks in systems comprising connected processes is known for example from US 2013/0260419 A1. As described above, in a sensor-controlled system according to an example of the invention, one or more separation stages or modules can be actively connected or disconnected (i.e. added or removed) based on the signals received from at least one sensor measuring at least one process and/or product property.

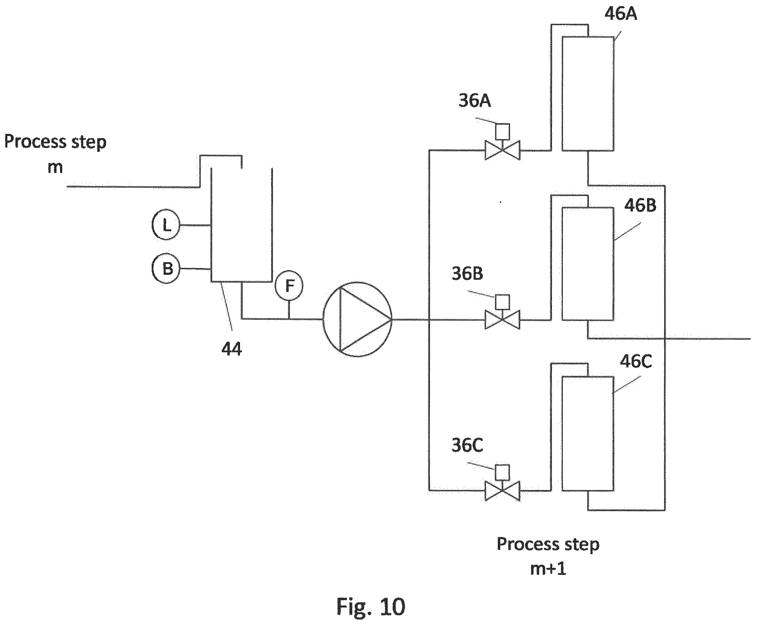

**Fig. 10** shows a block-diagram of an exemplary purification process using a system comprising an intermediate storage tank 44 for temporary storage of the output product of an "m"-th process step (e.g. m-th downstream processing step) prior to feeding it to a "m+1"-th process step.

Sensor L measures the level of liquid as indicator of the volume, sensor B measures the biomass in the intermediate storage tank 44. Sensor F arranged at the output of the intermediate storage tank 44 measures the flow as indicator of the volume flow rate. A group of downstream process stages 46A - 46C constituting the "m+1" process step is connected to the output of the intermediate storage tank 44 via respective actuators (e.g. valves) 36A - 36C. The actuators 36A - 36C are connected to and controlled by a control unit (not shown), such as the control unit 32 described above.

The system may, for example, be used as follows:
The product output from the m-th process stage is stored in an intermediate storage tank (intermediate storage stage). At least one product and/or process property is sensed or measured by at least one suitable sensor (e.g. sensors L, B and F). The at least one sensor may measure at least one feature in the intermediate storage tank 44, at the output or at the input of the intermediate storage tank 44. The at least one measured feature may be for example the volume and/or concentration of the product in the intermediate storage tank 44, and/or the concentration of at least one contaminant of the product in the intermediate storage tank 44, the amount of particles of the product in the intermediate storage tank 44, etc. Based on at least one measured feature the next process stage (process stage m + 1) may be configured and/or controlled by the control unit.

In particular, the m+1-th process step may be a group of "n" connected process stages or modules 46A - 44C, such as for example "n" purification stages or modules. The control unit may determine, based on the at least one measured feature the number of needed process stages or modules (such as the number of purification stages or modules) and may send control signals to respective actuators 36A - 36 C to connect (add) or disconnect (remove) the determined number of individual process stages or modules (such as purification stages or modules). The at least one measured feature may be, for example, the concentration of a product and/or contaminants, and/or particle properties and/or amount, etc. in or at the output of the intermediate storage tank 44.

In an example, the m+1-th process step performed in the m+1-th process stage may be started, only when the at least one measured value of the at least one feature reaches a predetermined value or is within a predetermined range. For example, the m+1-th process stage is only started when the amount of product from the process step "m" in the intermediate storage tank 44 reaches a predetermined value or is within a predetermined range. The available amount of product in the intermediate storage tank 44 may be determined based on the at least one measured (product) features.

In the above example, in addition or alternatively to the above mentioned upstream and/or downstream features, one or more of the following features may be measured and the measured values used by the control unit to configure, setup and/or control the process steps m and m+1 and the respective process stages. Typical measured features include:
Upstream: Volume, Viscosity (typically in the range of 1 to 5 cP), amount and/or composition of impurities (e.g. fraction of DNA, proteins, aggregates, etc.), product concentration (e.g. in the range of 1 to 200 g/L), turbidity.

Typical monitored disturbance features include deviations of the concentration of the target molecules or other target substances, of the amount and/or composition of impurities in intermediate and/or end products, of the temperature, of the filter performance (e.g. binding capacity, Flow performance, etc.), of the product concentration, etc.

The above examples relate to automatic setup, configuration and/or control of individual process stages, such as for example to automatic connection and disconnection of individual purification stages in a downstream process. Of course, the above described principles may be applied at any level of process granularity, such as for example to the automatic setup, configuration and/or control of the individual processing sub-stages of a particular process stage. Similarly, the above principles may be applied to the automatic setup, configuration and/or control of individual groups of process stages. Further, the number of process stages and steps is not limited to the number shown in the figures and may be higher or lower.. Similarly, the above principles are applicable not only to types of process stages described above, but also to other types of process stages or operations, such as for example to upstream process stages or operations and/or their substages.

The computational aspects described here can be implemented in digital electronic circuitry, or in computer hardware, firmware, software, or in combinations of them. When appropriate, aspects of these systems and techniques can be implemented in a computer program product, for example tangibly embodied in a machine-readable storage device for execution by a programmable processor; and method steps can be performed by a programmable processor executing a program of instructions to perform functions by operating on input data and generating an output.

To provide for interaction with a user, a computer system can be used having a display device, such as a monitor or a LCD screen for displaying information to the user and a keyboard, a pointing device such as a mouse or a trackball, a touch-sensitive screen, or any other device by which the user may provide input to computer system. The computer system can be programmed to provide a graphical user interface through which the computer program(s) interact(s) with the user.

A number of embodiments and examples have been described. Nevertheless, it will be understood that various modifications may be made. For example, the steps described can be performed in a different order and still achieve desirable results. Further, individual features of different embodiments and examples may be combined. Accordingly, other embodiments are within the scope of the claims.

Further, unless otherwise defined, all terms (including technical and scientific terms) used herein have the same meaning as commonly understood by one of ordinary skill in the art to which this invention belongs.

Further, the present disclosure relates to the following items and embodiments of the invention:
1. A computer-implemented method for configuring and/or setup and/or controlling of a downstream process for processing a biomass, comprising:
   receiving at least one measurement value of at least one feature (22) of an upstream process by which the biomass to be processed in the downstream process has been obtained and/or of at least one feature of a downstream process stage or operation (12, 18, 34A-34D, 38A-38D, 40, 42A-42D, 44; 120, 181-186);
   determining at least one downstream process parameter (20) based on the received at least one measurement value; and
   configuring and/or setup and/or controlling at least one downstream process stage or process operation (12, 18, 34A-34D, 38A-38D, 40, 42A-42D, 44; 120, 181-186) based on the determined at least one downstream process parameter (20).
2. The method according to item 1, wherein the at least one feature (22) includes a viable cell density, biomass, capacitance, cell viability and/or turbidity, and wherein the determining of the at least one downstream process parameter (20) comprises:
   selecting a purification stage or operation (34A-34D, 38A-38D, 40, 42A-42D);
   determining, based on the received at least one measurement value of the viable cell density, biomass, capacitance and/or cell viability, at least one process parameter of the selected purification stage or operation (34A-34D, 38A-38D, 40, 42A-42D) and/or determining a biomass dilution parameter.
3. The method according to item 2, wherein:
   wherein selecting a purification stage or operation includes selecting between a filter stage or operation and a centrifugal stage or operation, wherein:
   if a filter stage or operation is selected, the at least one process parameter includes at least one of the following parameters: a type of the filter stage or operation, number of filters, filter area, pressure, flow rate amount of diatomaceous earth and process time;
   if the centrifugal stage or operation is selected, the at least one process parameter includes the process time, process volume and centrifugal force.
4. The method according to any one of the preceding items, wherein the at least one feature includes titer, and wherein the determining of the at least one downstream process parameter (20) comprises determining, based on the received measurement value of the titer, at least one process parameter of a chromatography stage or operation, said at least one process parameter including one or more of a dimensioning, loading volume, cycle time, flow rate and number of chromatography columns.
5. The method according to any one of the preceding items, wherein the at least one feature (22) includes a host cell protein and/or DNA concentration and wherein the determining of the at least one process parameter (20) comprises determining of the at least one process parameter of an anion or a cation exchange chromatography stage or operation based on the received measurement value of the host cell protein and/or DNA concentration, said at least one process parameter including one or more of a volume of the chromatography column and/or membrane adsorber, buffer volume, number of cycles, and process time.
6. The method according to any one of the preceding items, wherein the at least one feature (22) includes the type and/or amount of at least one contaminant, and wherein the determining of the at least one downstream process parameter (20) comprises determining, based on the received measurement value of the type and/or amount of at least one contaminant, a biomass dilution parameter, cycle time and/or processing column size.
7. The method according to item 6, wherein the at least one feature includes the amount of antifoam and/or amount of block polymers.
8. The method according to any one of the preceding items, wherein the at least one feature (22) includes the pH value and/or the amount of at least one pH correctant, and wherein the determining of the at least one downstream process parameter (20) comprises determining, based on the received measurement value of the pH value and/or the amount of at least one pH correctant, the amount and/or type of at least one pH correctant for use in the downstream process.
9. The method according to any one of the preceding items,
   wherein the at least one feature (22) includes the amount of lipids and/or long epidermal growth factor, and/or peptides and/or other media, and wherein the determining of the at least one downstream process parameter (20) comprises determining, based on the received measurement value of the amount of lipids and/or long epidermal growth factor and/or peptides and/or other media, at least one purification operation parameter; and/or
   wherein the at least one feature (22) includes at least one critical quality attribute, and wherein the determining of the at least one downstream process parameter (20) comprises determining, based on the received measurement value of the at least one critical quality attribute, whether to start a downstream process; and/or
   wherein the at least one feature (22) includes the process temperature, and wherein the determining of the at least one downstream process parameter (20) comprises determining, based on the received process temperature, a cooling or heating of process fluid for the downstream process; and/or
   wherein the at least one feature (22) includes turbidity, and wherein the determining at least one downstream process parameter (20) comprises determining, based on the received measurement value of the turbidity, at least one purification operation parameter; and/or
   wherein the at least one feature (22) includes media buffer system and/or capacity, and wherein the determining of the at least one downstream process parameter (20) comprises determining, based on the received measurement value of the media buffer system and/or capacity, a pH adjustment parameter; and/or
   wherein the at least one feature (22) includes process volume, and wherein the determining of the at least one downstream process parameter (20) comprises determining, based on the received measurement value of the process volume, dimensioning of the downstream process.
10. The method according to any one of the preceding items, wherein said receiving comprises receiving a plurality of measurement values of respective plurality of process features (22) of an upstream process by which the biomass to be processed in the downstream process has been obtained and/or of a downstream process stage or operation.
11. The method according to any one of the preceding items, wherein
   the downstream process comprises a plurality of downstream process stages or operations that are connectable in parallel and/or sequentially (34A-34D, 38A-38D, 40, 42A-42);
   the at least one process parameter (20) includes the number and/or type of downstream process stages or operations to be connected or disconnected; and
   the configuring and/or setup and/or controlling comprises connecting or disconnecting the determined number and/or type of downstream process stages.
12. The method according to item 10 or 11, wherein the downstream process stage or operation to be connected or disconnected is a purification stage or operation (34A-34D, 38A-38C, 40, 42A-42C), and wherein optionally said purification stage or operation is one of a filtration stage, pre-filtration stage, chromatography stage or centrifugation stage._
13. The method according to any one of items 10 to 12, wherein the at least one process parameter (20) further includes at least one parameter characterizing the dimensioning of the determined number and/or type of downstream process stages or operations (34A-34D, 38A-38D, 40, 42A-42D).
14. The method according to any one of items 10 to 13, wherein the at least one feature (22) includes at least one of the following features: pressure, volume flow, volume flow rate, turbidity, viscosity, product amount and/or concentration, amount and/or concentration of contaminants, cell viability, temperature.
15. A computer program product comprising instructions which, when the program is executed by a computer, cause the computer to carry out the method of any one of the preceding items.
16. A system (10) for configuring and/or setup and/or controlling a downstream process for processing a biomass to obtain a product, said system comprising at least one processor (104) and at least one actuator (36A- 36D) controlled by the processor configured to carry out the method of any one of items 1 to 14.
17. A method for processing a biomass comprising:
   configuring and/or setup and/or controlling of a downstream process for processing the biomass according to the method of any one of items 1 to 14;
   processing the biomass by using the so configured and/or setup and/or controlled downstream process.
18. The method according to item 17, further comprising:
   performing an upstream process thereby obtaining the biomass to be processed in the downstream process;
   measuring at least one feature of the upstream and/or the downstream process.
19. A system (100) configured to process a biomass, said system comprising:
   a system (10) for configuring and/or setup and/or controlling a downstream process according to item 16, said system comprising at least one processor (104) configured to perform a method for configuring and/or setup of a downstream process for processing the biomass according to any one of items 1 to 14;
   at least one process stage (12, 18, 34A-34D, 38A-38D, 40, 42A-42D, 44; 120, 181-186) configured to process the biomass by using the so configured and/or setup and/or controlled downstream process.
20. The system (100) of item 19, further comprising
   at least one process stage configured to carry out an upstream process to thereby obtain the biomass to be processed in the downstream process;
   at least one measuring device for online or offline measuring of at least one feature of the upstream process.

### List of Reference Numerals

- 10: system for configuring and/or setup and/or control of a downstream process for processing a biomass
- 102: receiving device
- 104: process/processor system
- 106: database
- 12: upstream measurement values
- 14: communication network (e.g. computer network)
- 16: upstream process parameter
- 18: downstream purification stage
- 20: downstream process parameter
- 22: measured upstream and/or downstream features
- 30: bioreactor
- 32: control unit
- 34A- 34D: purification stages (e.g. filtration stage)
- 36A - 36D: actuators (e.g. valves)
- 38A - 38C: pre-purification stages (e.g. pre-filter stages)
- 40: main purification stage (e.g. main filter stage)
- 42A-42C: membrane absorbers
- 44: intermediate storage tank
- W: weight sensor (e.g. weight scale)
- B: biomass sensor
- P, P1, P2: pressure sensors
- F: flow sensor
- UV: UV light sensor e.g. at a wavelength of 230 - 300 nm
- L: level sensor
- 100: system for processing a biomass
- 100A: upstream processing sub-system;
- 100B: downstream processing sub-system;
- 110: bioreactor

- 120: protein A chromatography stage
- 140: communication network (e.g. computer network)
- 160: process relevant data
- 180: virus inactivation stage
- 181: stirring stage
- 182: low pH virus inactivating stage
- 183: stirring stage
- 184, 185: filtering stage
- 186: stirring stage

## Claims

1. A computer-implemented method for configuring and/or setup and/or controlling of a downstream process for processing a biomass, comprising:
receiving at least one measurement value of at least one feature (22) of an upstream process by which the biomass to be processed in the downstream process has been obtained and/or of at least one feature of a downstream process stage or operation (12, 18, 34A-34D, 38A-38D, 40, 42A-42C, 44; 120, 181-186);
determining at least one downstream process parameter (20) based on the received at least one measurement value; and
configuring and/or setup and/or controlling at least one downstream process stage or process operation (12, 18, 34A-34D, 38A-38D, 40, 42A-42C, 44; 120, 181-186) based on the determined at least one downstream process parameter (20),
wherein the at least one feature includes titer, and wherein the determining of the at least one downstream process parameter (20) comprises determining, based on the received measurement value of the titer, at least one process parameter of a chromatography stage or operation, said at least one process parameter including one or more of a dimensioning, loading volume, cycle time, flow rate and number of chromatography columns.

2. The method according to claim 1, wherein the downstream process is a batch process, the chromatography stage or operation comprises at least one chromatography column, and wherein determining of the at least one downstream process parameter (20) comprises:
determining an overall amount of the product to be processed in the chromatography stage or operation based on the measurement value of the titer, and
based on the determined overall amount of product to be processed,
i) determining and setting one or more of one of the dimensioning, loading volume, cycle time and/or flow rate of the at least one chromatography column required for processing the product within a predetermined time; or
ii) for a given dimension of the at least one chromatography column, determining and setting the processing time of the at least one chromatography column and/or the puffer amount for the at least one chromatography column.

3. The method according to claim 1, wherein the downstream process is a continuous process, the chromatography stage or operation comprises at least one chromatography column, wherein the at least one feature further includes flow rate or volume flow rate and wherein determining of the at least one downstream process parameter (20) comprises:
based on the titer and a volume flow or volume flow rate, i) determining, one or more of a number of chromatography columns necessary to ensure continuous flow and processing of the biomass; and/or ii) determining a number of cycles and/or the timing of a chromatography column(s) exchange; and/or iii) determining and/or adjusting at least one recipe of the downstream process, wherein optionally said determining and/or adjusting at least one recipe comprises setting or adjusting a loading volume and/or a volume flow rate.

4. The method according to any one of the preceding claims, wherein the at least one feature (22) further includes a viable cell density, biomass, capacitance, cell viability and/or turbidity, and wherein the determining of the at least one downstream process parameter (20) comprises:
selecting a purification stage or operation (34A-34D, 38A-38D, 40, 42A-42C);
determining, based on the received at least one measurement value of the viable cell density, biomass, capacitance and/or cell viability, at least one process parameter of the selected purification stage or operation (34A-34D, 38A-38D, 40, 42A-42C) and/or determining a biomass dilution parameter.

5. The method according to claim 4, wherein:
wherein selecting a purification stage or operation includes selecting between a filter stage or operation and a centrifugal stage or operation, wherein:
if a filter stage or operation is selected, the at least one process parameter further includes at least one of the following parameters: a type of the filter stage or operation, number of filters, filter area, pressure, flow rate amount of diatomaceous earth and process time;
if the centrifugal stage or operation is selected, the at least one process parameter further includes the process time, process volume and centrifugal force.

6. The method according to any one of the preceding claims, wherein
the at least one feature (22) further includes a host cell protein and/or DNA concentration and wherein the determining of the at least one process parameter (20) further comprises determining of the at least one process parameter of an anion or a cation exchange chromatography stage or operation based on the received measurement value of the host cell protein and/or DNA concentration, said at least one process parameter including one or more of a volume of the chromatography column and/or membrane adsorber, buffer volume, number of cycles, and process time; and/or
the at least one feature (22) further includes the pH value and/or the amount of at least one pH correctant, and wherein the determining of the at least one downstream process parameter (20) further comprises determining, based on the received measurement value of the pH value and/or the amount of at least one pH correctant, the amount and/or type of at least one pH correctant for use in the downstream process; and/or
wherein the at least one feature (22) further includes the type and/or amount of at least one contaminant, and wherein the determining of the at least one downstream process parameter (20) further comprises determining, based on the received measurement value of the type and/or amount of at least one contaminant, a biomass dilution parameter, cycle time and/or processing column size, and wherein optionally the at least one feature further includes the amount of antifoam and/or amount of block polymers.

7. The method according to any one of the preceding claims,
wherein the at least one feature (22) further includes the amount of lipids and/or long epidermal growth factor, and/or peptides and/or other media, and wherein the determining of the at least one downstream process parameter (20) further comprises determining, based on the received measurement value of the amount of lipids and/or long epidermal growth factor and/or peptides and/or other media, at least one purification operation parameter; and/or
wherein the at least one feature (22) further includes at least one critical quality attribute, and wherein the determining of the at least one downstream process parameter (20) further comprises determining, based on the received measurement value of the at least one critical quality attribute, whether to start a downstream process; and/or
wherein the at least one feature (22) includes further the process temperature, and wherein the determining of the at least one downstream process parameter (20) further comprises determining, based on the received process temperature, a cooling or heating of process fluid for the downstream process; and/or
wherein the at least one feature (22) includes further turbidity, and wherein the determining at least one downstream process parameter (20) further comprises determining, based on the received measurement value of the turbidity, at least one purification operation parameter; and/or
wherein the at least one feature (22) further includes media buffer system and/or capacity, and wherein the determining of the at least one downstream process parameter (20) further comprises determining, based on the received measurement value of the media buffer system and/or capacity, a pH adjustment parameter; and/or
wherein the at least one feature (22) further includes process volume, and wherein the determining of the at least one downstream process parameter (20) further comprises determining, based on the received measurement value of the process volume, dimensioning of the downstream process.

8. The method according to any one of the preceding claims, wherein said receiving comprises receiving a plurality of measurement values of respective plurality of process features (22) of an upstream process by which the biomass to be processed in the downstream process has been obtained and/or of a downstream process stage or operation.

9. The method according to any one of the preceding claims, wherein
the downstream process comprises a plurality of downstream process stages or operations that are connectable in parallel and/or sequentially (34A-34D, 38A-38D, 40, 42A-42C), the at least one process parameter (20) further includes the number and/or type of downstream process stages or operations to be connected or disconnected, and the configuring and/or setup and/or controlling comprises connecting or disconnecting the determined number and/or type of downstream process stages.

10. The method according to claim 9, wherein the downstream process stage or operation to be connected or disconnected is a purification stage or operation (34A-34D, 38A-38C, 40, 42A-42C), and wherein optionally said purification stage or operation is one of a filtration stage, pre-filtration stage, chromatography stage or centrifugation stage._

11. The method according to claim 9 or 10,
wherein the at least one process parameter (20) further includes at least one parameter characterizing the dimensioning of the determined number and/or type of downstream process stages or operations (34A-34D, 38A-38D, 40, 42A-42C); and/or
wherein the at least one feature (22) includes at least one of the following features: pressure, volume flow, volume flow rate, turbidity, viscosity, product amount and/or concentration, amount and/or concentration of contaminants, cell viability, temperature.

12. A computer program product comprising instructions which, when the program is executed by a computer, cause the computer to carry out the method of any one of the preceding claims.

13. A system (10) for configuring and/or setup and/or controlling a downstream process for processing a biomass to obtain a product, said system comprising at least one processor (104) and at least one actuator (36A- 36D) controlled by the processor configured to carry out the method of any one of claims 1 to 11.

14. A method for processing a biomass comprising:
configuring and/or setup and/or controlling of a downstream process for processing the biomass according to the method of any one of claims 1 to 11;
processing the biomass by using the so configured and/or setup and/or controlled downstream process.

15. A system (100) configured to process a biomass, said system comprising:
a system (10) for configuring and/or setup and/or controlling a downstream process according to claim 16, said system comprising at least one processor (104) configured to perform a method for configuring and/or setup of a downstream process for processing the biomass according to any one of claims 1 to 11;
at least one process stage (12, 18, 34A-34D, 38A-38D, 40, 42A-42C, 44; 120, 181-186) configured to process the biomass by using the so configured and/or setup and/or controlled downstream process.
